# EUROPEAN PATENT APPLICATION

(11) **EP 4 725 944 A1**
(43) Date of publication of application: **15.04.2026**
(21) Application number: 24818802.1
(22) Date of filing: 07.06.2024
(51) Int. Cl.: C07D 401/10, C07C 303/28, C07C 309/73, A61P 9/10, A61P 7/02

(54) **NEW METHOD FOR PREPARING OXOPYRIDINE COMPOUND, AND KEY INTERMEDIATE AND USE**

(30) Priority: 09.06.2023 CN 202310679927; 21.06.2023 CN 202310738580; 26.06.2023 CN 202310757873
(71) Applicant: Chengdu Shibeikang Biomedical Technology Co., Ltd., Chengdu, Sichuan 611731 (CN)
(72) Inventor: ZHOU, Guanglin, Chengdu, Sichuan 611731 (CN); HUANG, Long, Chengdu, Sichuan 611731 (CN); ZHU, Xucheng, Chengdu, Sichuan 611731 (CN); ZENG, Yanqun, Chengdu, Sichuan 611731 (CN)
(74) Representative: IP TRUST SERVICES
(86) International application number: PCT/CN2024/098225
(87) International publication number: WO 2024/251276

(57) **Abstract**

A new route for preparing an oxopyridine compound as represented by formula (I) and a key intermediate thereof. The new route can greatly reduce the generation of isomer impurities, improve the selectivity of the reaction chirality and the selectivity of N/O-alkylation, increase the yield, avoid re-purification of a crude product, and reduce the cost, has a short production period, and is energy-saving and environmentally-friendly, and suitable for preparing a drug for treating and/or preventing diseases related to FXIa receptors, and particularly provides a new idea for preparing a drug for treating and/or preventing cerebrovascular artery diseases and/or peripheral artery diseases.

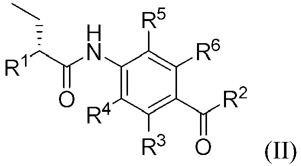

## Description

### TECHNICAL FIELD

The invention belongs to the field of medicinal chemistry and preparation processes, and specifically relates to a novel method for preparing an oxopyridine compound, key intermediates, and uses thereof.

### BACKGROUND

Thromboembolic disorders are diseases in humans and animals caused by abnormal intravascular clot formation in vivo. Coagulation factor XI (FXI) is a plasma serine protease zymogen essential for the intrinsic pathway. Upon activation, it generates activated factor XIa (FXIa), which plays a pivotal role in the amplification of the coagulation cascade. Thrombin provides feedback for FXI, while activated FXIa in turn drives substantial thrombin generation, thereby amplifying the coagulation cascade. Consequently, FXIa-targeted agents can interrupt the intrinsic pathway and attenuate cascade amplification, providing antithrombotic activity. Recent research indicates that the inhibition of FXIa may carry a lower risk of bleeding than direct FXa inhibitors, making FXIa a promising target for antithrombotic prophylaxis and treatment. Among them, Bayer's anticoagulant BAY-2433334 has garnered considerable interest in the field owing to less bleeding.

Regarding anticoagulant BAY-2433334, Bayer Pharmaceutical's compound patent CN108026072B highlights two oxopyridine compounds, as follows:

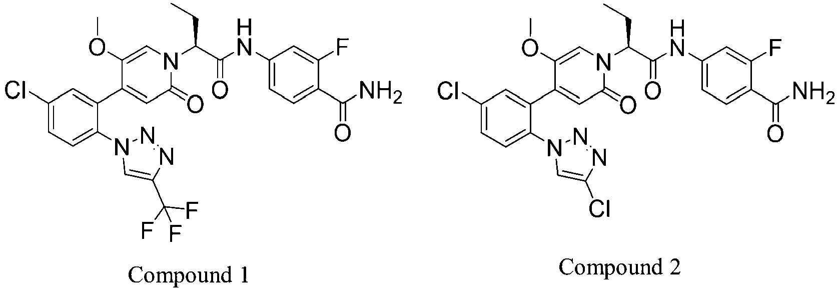

Such molecules are structurally complex, difficult to synthesize , and their isomers are difficult to separate, making scale-up particularly challenging. Chengdu Shibeikang has undertaken extensive structural optimization and process development, aiming to provide species with improved performance and better suitability for industrial-scale manufacturing.

In particular, in studies of the preparation of the anticoagulant derivatives of Formula (I) below, the methods disclosed in Bayer Pharmaceutical's patents of compound and process could not afford the active pharmaceutical ingredients in high yield and high purity. 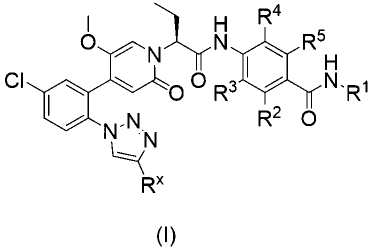
R^{x} is selected from fluoro, chloro or trifluoromethyl;
R¹ is selected from alkyl or deuterated methyl;
R², R³, R⁴, and R⁵ are independently selected from hydrogen, halogen, alkoxy, or
haloalkyl.

Application publication WO 2014/154794 and WO 2017/005725 describe the synthesis of such compounds. The process uses 2,5-dimethoxypyridine as the starting material, and employs a nine-step linear synthetic strategy for target compound. The route is lengthy, and prone to high racemization, resulting in a low overall yield. At the crude synthetic step, the yield is only 70%. Complex post-processing and purification procedures are required, and isomers are separated by HPLC or chiral supercritical fluid chromatography (SFC). The process is time-consuming and costly, and is not suitable for industrial scale-up.

Application publication CN 111770917 A discloses a convergent synthetic strategy, and the steps for obtaining the crude products are as shown as below, wherein the key intermediates, Formula (XVI-CF3)/(XVI-Cl) and Formula (XIX), are prepared separately, and are then condensed to afford the crude products of Compounds 1/2. The reaction comprises six steps overall with the longest linear sequence comprising only four steps, thereby shortening the reaction time. Enantioselectivity and N/O-alkylation selectivity are specifically optimized in the crude-product formation step. After filtration and evaporation of solvent, the condensation product affords amorphous crude Compounds 1/2 with high ee (85-93% ee). Further, a preferred N-alkylation over the undesired O-alkylation is obtained, with an N-alkylation:O-alkylation ratio of 9:1 to 10:1.

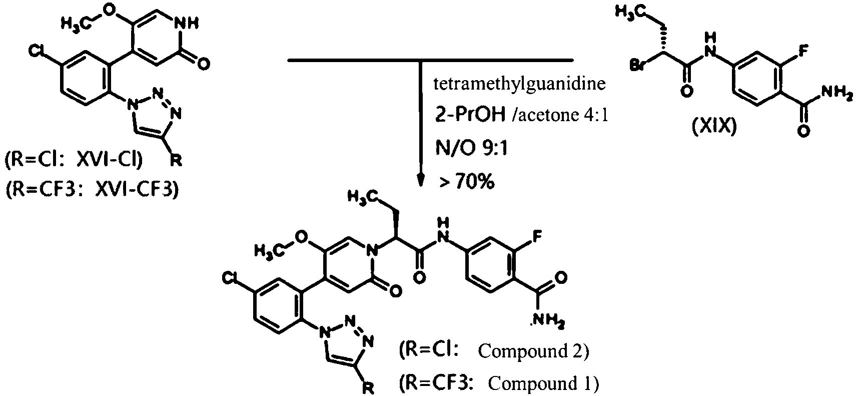

Although the convergent synthetic route therein is overall superior to a linear synthesis strategy, the condensation step used to generate the crude product still has significant limitations, such as: (1) Low conversion, the condensation step affords only 70% and 75% yields for the crude syntheses of Compounds 1 and 2, respectively , an alternative protocol affords only 61% (c.f. Specification, [0095]-[0097]); and the cumulative yield over the six-step is only 20%-25% (c.f. Specification [0054]); (2) The proportion of isomers is relatively high; although the crude has been optimized to 85-93% ee, 7-15% isomeric impurities still remain. Additional purification with an organic solvent is required to obtain a purified crude with >99% ee, followed by crystallization to obtain a target crystalline form; (3)N/O-alkylation selectivity is poor; although the N-alkylation:O-alkylation ratio of crude products reaches (9-10):1, 10% undesired O/N conversion remains. This not only results in low conversion rate but also generates a considerable amount of O-alkylated impurities, increasing the difficulty of downstream purification and and the risk of product quality-control.

Therefore, enhancing the quality of oxopyridinone-based anticoagulant products, reducing impurity-control risks, increasing product conversion and purity, shortening the production cycle, lowering costs, and ensuring suitability for industrial scale-up remain urgent technical challenges in this field.

### SUMMARY

To solve the technical problems in the prior art, the present invention discloses a novel preparation method for oxopyridine compound along with key intermediates thereof and use thereof.

In one aspect, the present invention provides an intermediate of Formula (II) or a pharmaceutically acceptable salt thereof:

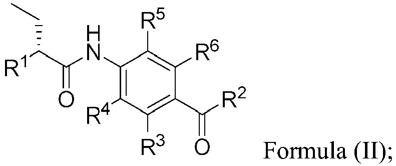

In Formula (II):
R¹ is selected from TsO-, X, R⁷O-; Ts is p-toluenesulfonyl, and X is selected from F, Cl, Br, or I;
R² is selected from -NHR⁸, -OC(CH₃)₃;
R³, R⁴, R⁵, and R⁶ are each independently selected from hydrogen, halogen, alkoxy, or haloalkyl;
R⁷ is selected from trifluoromethanesulfonyl, methanesulfonyl, p-nitrobenzenemethanesulfonyl or benzenesulfonyl;
R⁸ is selected from hydrogen, alkyl or cycloalkyl; 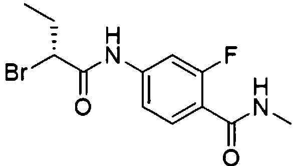
provided that Formula (II) is not

Further, the intermediate has the structure of Formula (III), (IV), or (V): 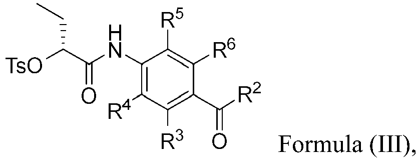 In Formula (III),
Ts is p-toluenesulfonyl;
R² is selected from -NHR⁸, and R⁸ is selected from hydrogen, alkyl or cycloalkyl;
R³, R⁴, R⁵, and R⁶ are independently selected from hydrogen, halogen, alkoxy, or haloalkyl; 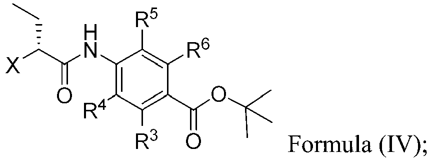 In Formula (IV):
   R³, R⁴, R⁵, and R⁶ are independently selected from hydrogen, halogen, alkoxy, or haloalkyl;
   X is selected form F, Cl, Br, or I; 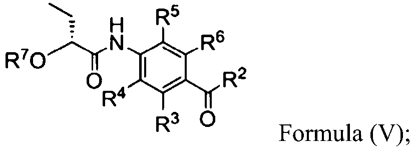 in Formula (V):
      R² is selected from NHR⁸, and R⁸ is selected from hydrogen, alkyl or cycloalkyl;
      R³, R⁴, R⁵, and R⁶ are each independently selected from hydrogen, halogen, alkoxy, or haloalkyl;
      R⁷ is selected from trifluoromethanesulfonyl, methanesulfonyl, p-nitrobenzenemethanesulfonyl or benzenesulfonyl.

Further, in the intermediate of Formula (III), or a pharmaceutically acceptable salt thereof:
R² is selected from -NHR⁸, and R⁸ is selected from hydrogen, methyl, ethyl, propyl, cyclopropyl, cyclopropylmethyl or tert-butyl;
and/or R³, R⁴, R⁵, and R⁶ are independently selected from hydrogen, fluoro, chloro, methoxy, ethoxy or trifluoromethyl.

Further, in the intermediate of Formula (IV) described above, or a pharmaceutically acceptable salt thereof:
and/or R³, R⁴, R⁵, and R⁶ are independently selected from hydrogen, fluoro, chloro, methoxy, ethoxy or trifluoromethyl.

Further, in the intermediate of Formula (V), or a pharmaceutically acceptable salt thereof:
R⁸ is selected from hydrogen, methyl, ethyl, propyl, cyclopropyl, cyclopropylmethyl or tert-butyl;
and/or R³, R⁴, R⁵, and R⁶ are independently selected from hydrogen, fluoro, chloro, methoxy, ethoxy or trifluoromethyl.

Further, in the intermediate of Formula (IV) described above, or a pharmaceutically acceptable salt thereof, the Formula (IV) intermediate comprises the structure of Formula (IV-a): 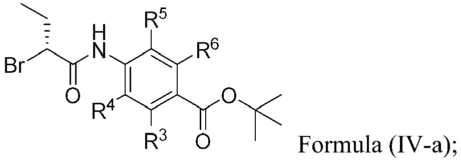 in Formula (IV-a), R³, R⁴, R⁵, and R⁶ are as previously defined.

Further, in any of the foregoing intermediates, at least of hydrogen atoms in the structure may be replaced with deuterium.

Further, the intermediate is selected from the group consisting of the following compounds:

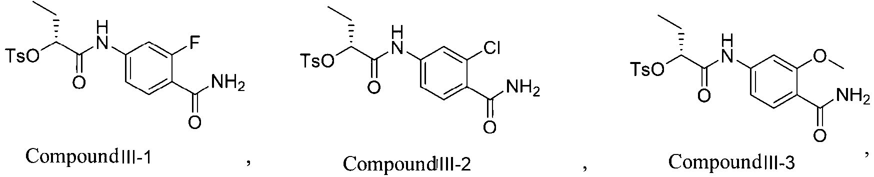

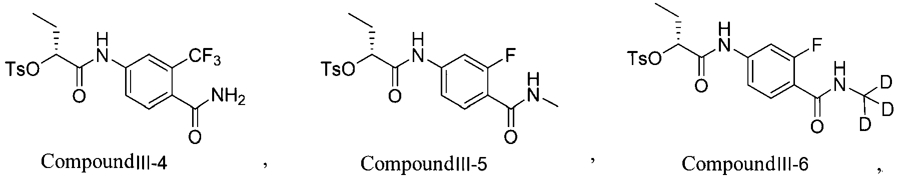

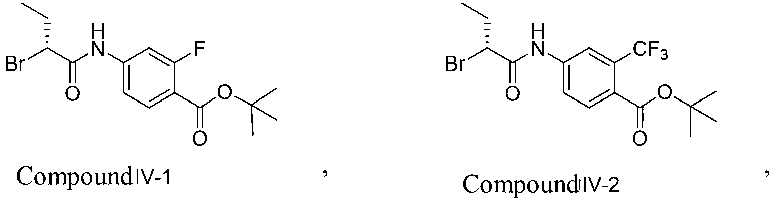

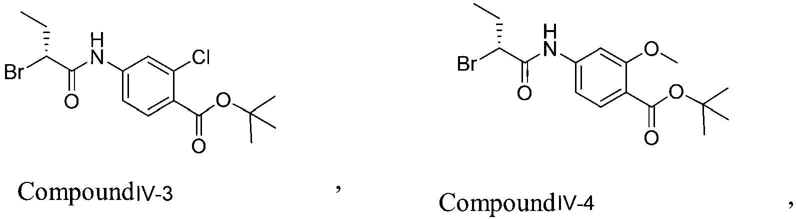

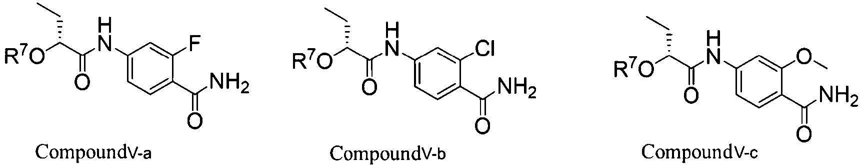

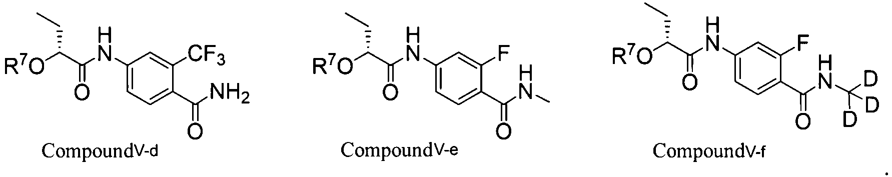

Further, the present invention provides a method for preparing the intermediate of Formula (II), or a pharmaceutically acceptable salt thereof, which comprises the following steps: 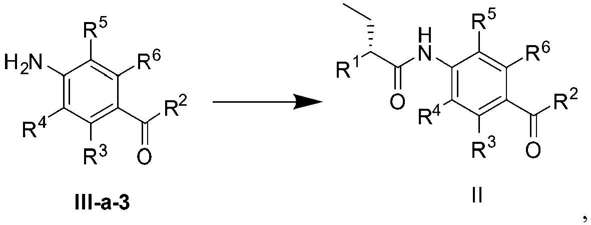 wherein, R¹, R², R³, R⁴, R⁵, and R⁶ are defined the same as in any of the foregoing definitions.

Further, the preparation method for preparing the intermediate of Formula (II) include method A, B, or C:

Method A: The compound of Formula (III-a) is reacted with p-toluenesulfonyl chloride under basic conditions to give the intermediate of Formula (III). 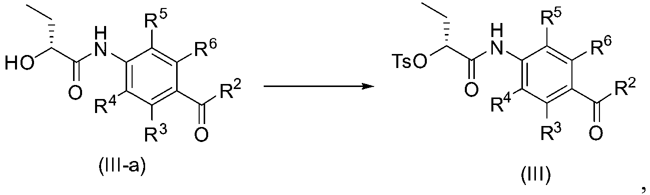 wherein, R², R³, R⁴, R⁵, and R⁶ are defined the same as in any of the foregoing definitions;

Method B: The compound of Formula (III-a-5) is reacted with halogenated carboxylic acid to give the intermediate of Formula (IV). 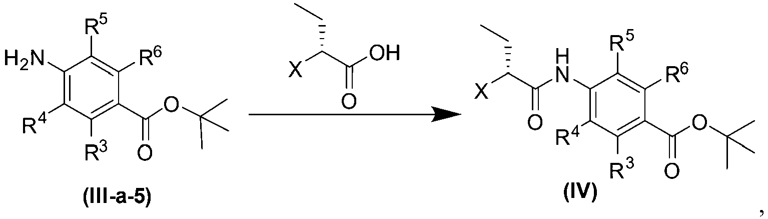 wherein, X is selected form F, Cl, Br or I; R³, R⁴, R⁵, and R⁶ are defined the same as in any of the foregoing definitions;

Method C: The compound of Formula (III-a) undergoes esterification with a hydroxyl-protecting reagent under basic conditions to give the intermediate of Formula (V). 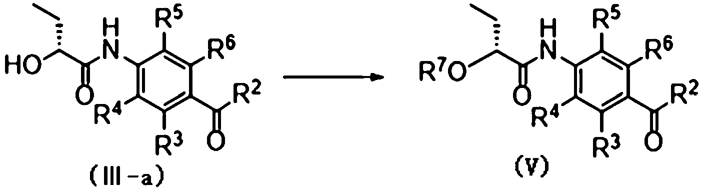 wherein, R², R³, R⁴, R⁵, R⁶, and R⁷ are defined the same as in any of the foregoing definitions.

Further, the halogenated carboxylic acid in Method B is selected from bromocarboxylic acids of the following structure:

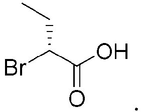

Further, the hydroxyl-protecting reagent in Method C comprises trifluoromethanesulfonyl chloride, methanesulfonyl chloride, p-nitrobenzenemethanesulfonyl chloride, and benzenesulfonyl chloride.

Moreover, Method A and/or Method C comprise the following reaction conditions:

The base is selected from organic bases; preferably, the organic base comprises triethylamine, pyridine, DMAP, tetramethylguanidine, DBU, or DIPEA, more preferably triethylamine or pyridine;
the reaction solvent in the synthetic step is selected from organic solvents; preferably, the organic solvent comprises, but is not limited to, any one of tetrahydrofuran, 2-methyltetrahydrofuran, isopropanol, ethanol, acetone, DMF, acetonitrile, dichloromethane, or a mixture of two or more thereof.

Optionally, the molar ratio of the compound of Formula (III-a) to the organic base in the synthesis step is 1:0.5-8, preferably 1:1-3.

Optionally, the reaction temperature of the synthesis step is 0-60 °C, preferably 10-30 °C;

Optionally, the reaction time of the synthesis step is 1-10 h, preferably 4-6 h.

Moreover, Method B described above comprises the following reaction conditions:

The reaction conditions comprise an organic base; preferably, the organic base comprises triethylamine, pyridine, DBU, or DIPEA;

The reaction conditions comprise an organic solvent; preferably, the organic solvent comprises tetrahydrofuran, 2-methyltetrahydrofuran, ethyl acetate, DMF, or acetonitrile;

The reaction conditions include a condensing agent; the condensing agent comprises 1-propylphosphoric anhydride, DCC, and EDCI.

The reaction temperature is -10-10 °C for 5-60 min, followed by 10-50 °C for 10-600 min; preferably, -0-5 °C for 10 min, and then at room temperature for 30 min.

Further, the method for preparing the compound of Formula (III-a) comprises the following steps:

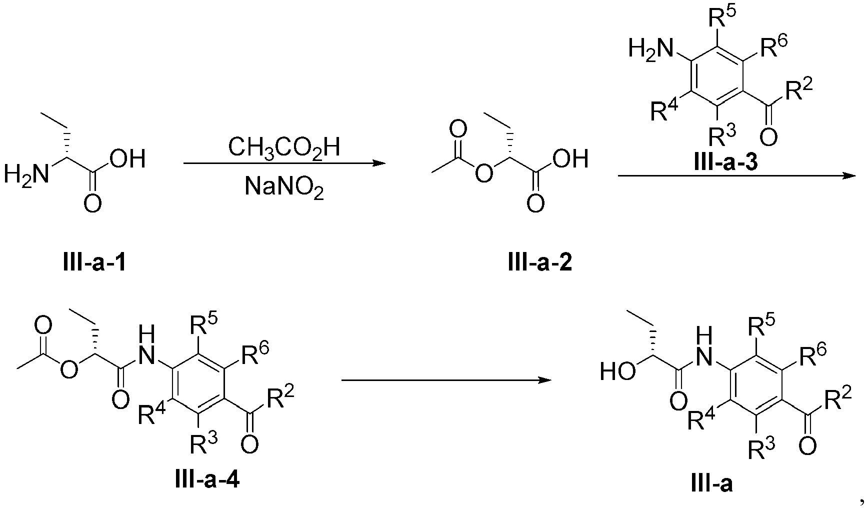

D-2-aminobutyric acid of Formula (III-a-1) is diazotized with sodium nitrite in acetic acid to afford the compound of Formula (III-a-2);

The compound (III-a-2) is subjected to an amide condensation reaction with the compound of Formula (III-a-3) in the presence of a base and an acid anhydride to afford the compound of Formula (III-a-4).

The intermediate (III-a-4) is then hydrolyzed to give the compound (III-a).

Further, in the step of preparing the compound of Formula (III-a-2), D-2-aminobutyric acid is dissolved in acetic acid; sodium nitrite is added at 0-5 °C, and the mixture is stirred at 0-5 °C for 6-10 h.

Optionally, the molar ratio of D-2-aminobutyric acid to sodium nitrite is 1:1.8-2.3, preferably 1:2.

Further, the amide condensation for preparing compound (III-a-4) from compound (III-a-2) comprises: combining compound (III-a-2) and compound (III-a-3) in a molar ratio of 1.3-1.8:1; adding a base and an acid anhydride at -10 -0 °C; and stirring the mixture at 0-5 °C for 5-15 min, followed by stirring at room temperature for 20-60 min.

Optionally, the molar ratio of the compound of Formula (III-a-2) to the compound of Formula (III-a-3) is 1.3-1.8-1, preferably 1.5:1.

Optionally, the reaction conditions comprise stirring at 0-5 °C for 5-13 min, followed by stirring at room temperature for 20-40 min.

In another aspect, the present invention the use of any intermediates, or pharmaceutically acceptable salts thereof, as standards or references, in the preparation of the oxopyridine compounds of Formula (I) , or in preparation of medicines for the treatment or prophylaxis of vascular and arterial diseases.

In another aspect, the invention provides a method for preparing the oxopyridine compounds of Formula (I), comprising reacting an intermediate of Formula (II) or a pharmaceutically acceptable salt thereof with a compound of Formula (VI) to afford a compound of Formula (I); 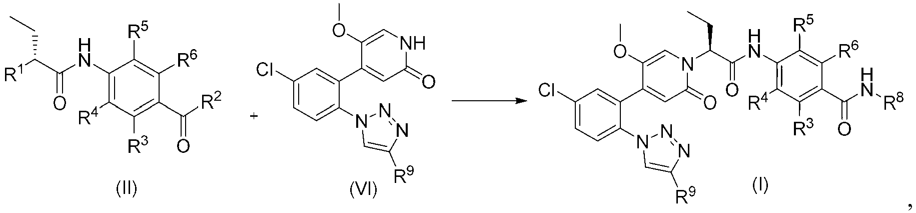 In Formula (II):
R¹ is selected from TsO-, X, R⁷O-; Ts is p-toluenesulfonyl, and X is selected from F, Cl, Br, or I;
R² is selected from -NHR⁸, -OC(CH₃)₃;
R³, R⁴, R⁵, and R⁶ are independently selected from hydrogen, halogen, alkoxy, or haloalkyl;
R⁷ is selected from trifluoromethanesulfonyl, methanesulfonyl, p-nitrobenzenemethanesulfonyl orbenzenesulfonyl;
R⁸ is selected from hydrogen, alkyl or cycloalkyl;
R⁹ is selected from fluoro, chloro or trifluoromethyl;
Provided that Formula (II) is not

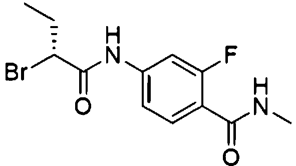

Further, the method for preparing the oxopyridine compound of Formula (I) comprises the following steps: the preparation method include:
when R² is selected from -OC(CH₃)₃, the intermediate of Formula (II) has the structure of Formula (II-a) and the preparation method comprises the following steps: 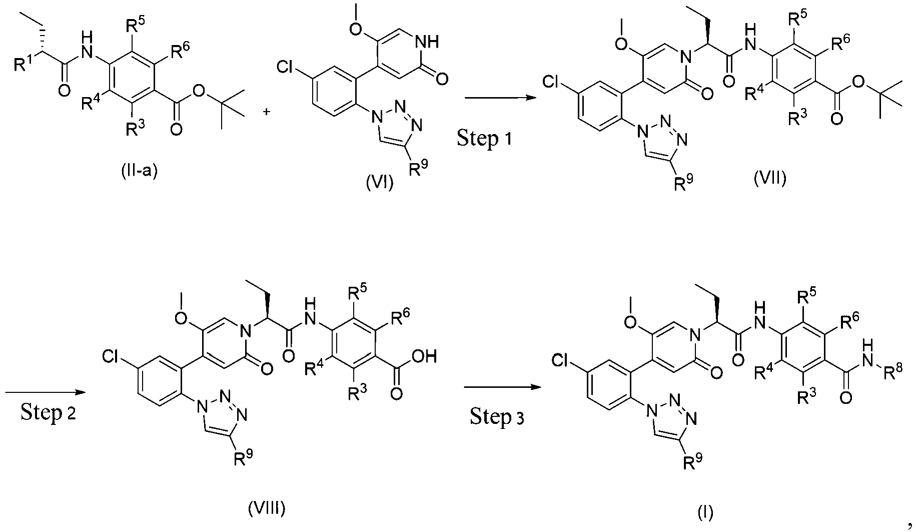 wherein,
R¹ is selected from TsO-, X, R⁷O-; Ts is p-toluenesulfonyl, and X is selected from F, Cl, Br, or I;
R³, R⁴, R⁵, and R⁶ are each independently selected from hydrogen, halogen, alkoxy, or haloalkyl;
R⁷ is selected from trifluoromethanesulfonyl, methanesulfonyl, p-nitrobenzenemethanesulfonyl or benzenesulfonyl;
R⁸ is selected from hydrogen, alkyl or cycloalkyl;
R⁹ is selected from fluoro, chloro or trifluoromethyl;
Step 1: the intermediate of Formula (II-a), or a pharmaceutically acceptable salt thereof, is reacted with the compound of Formula (VI) to give the compound of Formula (VII);
Step 2: the compound of Formula (VII) is hydrolyzed to give the compound of Formula (VIII);
Step 3: the compound of Formula (VIII) is subjected to condensation to afford the compound of Formula (I).

Further, the method for preparing the oxopyridine is selected from Method 1, 2 or 3;
Method 1: the preparation method comprises obtaining the compound of Formula (I) by the reaction of the intermediate of Formula (III), or a pharmaceutically acceptable salt thereof with the compound of Formula (VI), 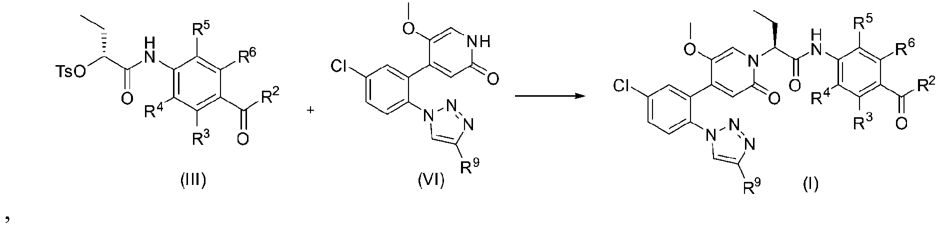 wherein,
   Ts is selected from p-toluenesulfonyl;
   R² is selected from -NHR⁸, and R⁸ is selected from hydrogen, alkyl or cycloalkyl;
   R³, R⁴, R⁵, and R⁶ are independently selected from hydrogen, halogen, alkoxy, or haloalkyl;
   R⁹ is selected from fluoro, chloro or trifluoromethyl;
Method 2: 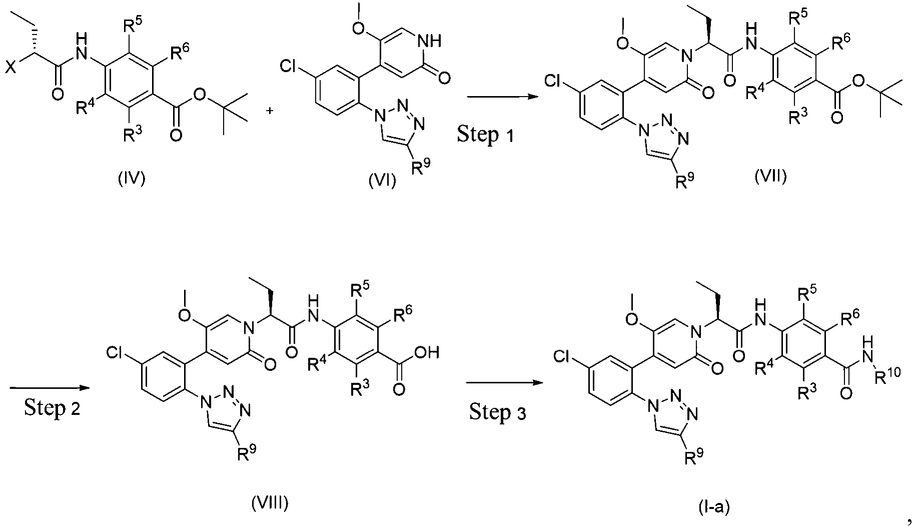 wherein,
   R¹⁰ is selected from alkyl, cycloalkyl, or deuterated alkyl, or cycloalkyl;
   R³, R⁴, R⁵, and R⁶ are independently selected from hydrogen, halogen, alkoxy, or haloalkyl;
   R⁹ is selected from fluoro, chloro or trifluoromethyl;
   X is selected form F, Cl, Br, or I;
   Step 1: the intermediate of Formula (IV), or a pharmaceutically acceptable salt thereof, is reacted with the compound of Formula (VI) to give the compound of Formula (VII);
   Step 2: the compound of Formula (VII) is hydrolyzed to give the compound of Formula (VIII);
   Step 3: the compound of Formula (VIII) is subjected to condensation to afford the compound of Formula (I-a).
Method 3:

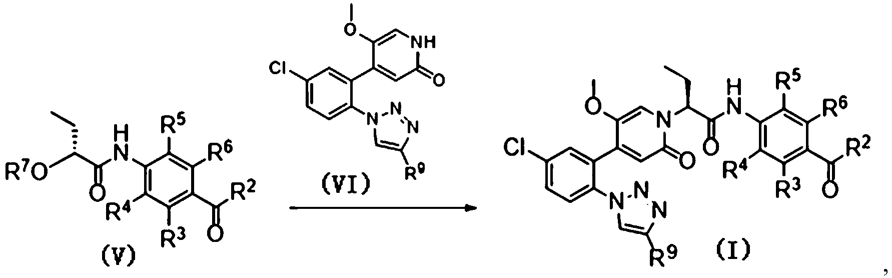

The intermediate of Formula (V) is reacted with the Formula (VI) compound under basic conditions via nucleophilic substitution to afford the Formula (I) compound;
wherein,
R⁹ is selected from fluoro, chloro or trifluoromethyl;
R² is selected from NHR⁸, and R⁸ is selected from hydrogen, alkyl or cycloalkyl;
R³, R⁴, R⁵, and R⁶ are independently selected from hydrogen, halogen, alkoxy, or haloalkyl;
R⁷ is selected from trifluoromethanesulfonyl, methanesulfonyl, p-nitrobenzenemethanesulfonyl or benzenesulfonyl.

Moreover, in Method 1 described above:
R⁹ is selected from fluoro, chloro or trifluoromethyl;
Ts is seleced from p-toluenesulfonyl;
R² is selected from -NHR⁸, wherein, R⁸ is selected from hydrogen, methyl, ethyl, propyl, cyclopropyl, cyclopropylmethyl or tert-butyl;
and/or R³, R⁴, R⁵, and R⁶ are independently selected from hydrogen, fluoro, chloro, methoxy, ethoxy or trifluoromethyl.

Moreover, Method 1 described above comprises the following reaction conditions:

Method 1 comprises a base; the base is selected from organic or inorganic bases. Preferably, the base includes any one of sodium carbonate, potassium carbonate, cesium carbonate, potassium bicarbonate, sodium bicarbonate, lithium hydroxide, sodium hydroxide, potassium hydroxide, tetramethylguanidine, triethylamine, DBU, DIPEA, and pyridine, or a mixture of two or more thereof. More preferably, the base includes any one of potassium carbonate, cesium carbonate, tetramethylguanidine, triethylamine, DBU, and DIPEA, or a mixture of two or more thereof.

The reaction solvent of Method 1 is selected from organic solvents; preferably, the organic solvents comprise any one of tetrahydrofuran, 2-methyltetrahydrofuran, isopropanol, ethanol, acetone, DMF, acetonitrile, ethyl acetate, or a mixture of two or more thereof.

Optionally, the molar ratio of the compound of Formula (III-a) to the organic base in the synthesis step is 1:1-3, preferably 1:1-2.

Optionally, the reaction temperature of Method 1 is 0-60 °C, preferably 20-40 °C, more preferably 26-32 °C.

Optionally, the reaction time of Method 1 is 1-10 h, preferably 4-6 h,

Further, the Method 1 described above comprises the synthetic steps of compound (III):
which is obtained by reaction of the compound of Formula (III-a) with p-toluenesulfonyl chloride under basic conditions: 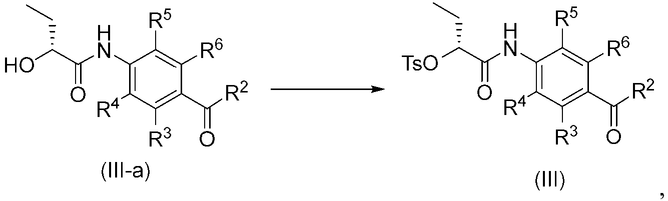
wherein, R², R³, R⁴, R⁵, and R⁶ are defined the same as in any of the foregoing definitions.

Further, the synthetic step of Formula (III) comprises the following steps:

The base is selected from organic bases; preferably, the organic base comprises triethylamine, pyridine, tetramethylguanidine, DMAP, DBU, or DIPEA, more preferably triethylamine or pyridine;
the reaction solvent in the synthetic step is selected from organic solvents; preferably, the organic solvent comprises, but is not limited to, any one of tetrahydrofuran, 2-methyltetrahydrofuran, isopropanol, ethanol, acetone, DMF, acetonitrile, dichloromethane, or a mixture of two or more thereof.

Optionally, the molar ratio of the compound of Formula (III-a) to the organic base in the synthesis step is 1:0.5-8, preferably 1:1-3.

Optionally, the reaction temperature of the synthesis step is 0-60 °C, preferably 10-30 °C;

Optionally, the reaction time of the synthesis step is 1-10 h, preferably 4-6 h.

Further, the method for preparing the compound of Formula (III-a) comprises the following steps: 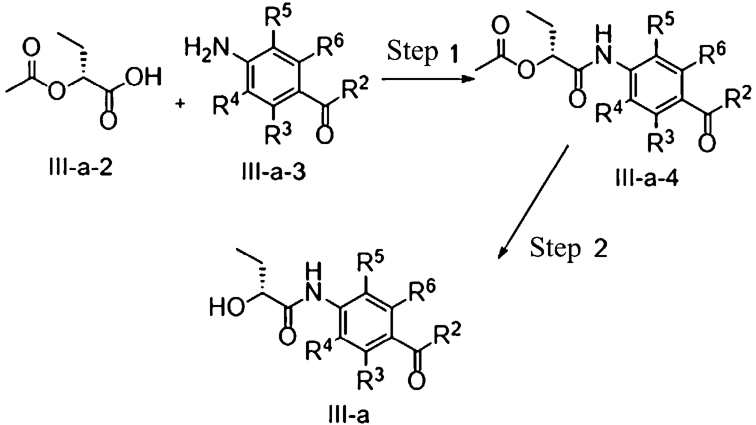 wherein, R², R³, R⁴, R⁵, and R⁶ are defined the same as in any of the foregoing definitions;
Step 1: The compound (III-a-2) is subjected to a condensation reaction with the compound of Formula (III-a-3) to afford the compound of Formula (III-a-4);
Step 2: the compound of Formula (III-a-4) is hydrolyzed under basic conditions to obtain the compound of formula (III-a).

Further, the condensation reaction in step 1 of the preparation of the (III-a) compound described above includes the following reaction conditions:
The condensation reaction conditions include a condensing agent; preferably, the condensing agent comprises T3P or DPP-Cl;
The condensation reaction conditions further include an organic base; preferably, the organic base comprises triethylamine, pyridine, tetramethylguanidine, DBU, or DIPEA, more preferably triethylamine or pyridine;
The reaction solvent is an organic solvent; preferably, the organic solvent comprises any one of tetrahydrofuran, 2-methyltetrahydrofuran, isopropanol, ethanol, acetone, DMF, acetonitrile, ethyl acetate, or a mixture of two or more thereof.

Optionally, the condensation reaction temperature is 0-60 °C, preferably 10-30 °C;
Optionally, the condensation reaction time is 1-10 h, preferably 2-4 h.

Further, the hydrolysis reaction in step 2 of the preparation of the (III-a) compound described above includes the following reaction conditions:
The hydrolysis reaction includes an inorganic base; preferably, the inorganic base includes potassium carbonate, sodium carbonate, cesium carbonate, potassium bicarbonate, or sodium bicarbonate; more preferably, potassium carbonate.

The solvent for the hydrolysis reaction is a mixture of an organic solvent and water. Preferably, the volume ratio of organic solvent to water in the mixed solvent is 1:1-10, more preferably 1:1-2. The mixed solvent includes methanol and water, ethanol and water, tetrahydrofuran and water, or DMSO and water, preferably methanol and water;
Optionally, the molar ratio of the compound of Formula (III-a-4) to the inorganic base in the hydrolysis reaction is 1:1-10, preferably 1:1-3.

Optionally, the hydrolysis reaction temperature is 0-60 °C, preferably 10-30 °C;
Optionally, the hydrolysis reaction time is 1-10 h, preferably 5-7 h.

Further, in Method 3, in the nucleophilic substitution reaction for preparing the compound of Formula (I) from the intermediate of Formula (V), the molar ratio of the compound of Formula (V) to the base is 1:1-3, the reaction temperature is 0-60 °C, and the reaction time is 1-10 h.

Moreover, Method 3 described above comprises the following reaction conditions:
Method 3 comprises a base; the base is selected from organic or inorganic bases. Preferably, the base includes any one of sodium carbonate, potassium carbonate, cesium carbonate, potassium bicarbonate, sodium bicarbonate, lithium hydroxide, sodium hydroxide, potassium hydroxide, tetramethylguanidine, triethylamine, DBU, DIPEA, and pyridine, or a mixture of two or more thereof. More preferably, the base includes any one of potassium carbonate, cesium carbonate, tetramethylguanidine, triethylamine, DBU, and DIPEA, or a mixture of two or more.

The reaction solvent of Method 3 is selected from organic solvents; preferably, the organic solvents comprise any one of tetrahydrofuran, 2-methyltetrahydrofuran, isopropanol, ethanol, acetone, DMF, acetonitrile, ethyl acetate, or a mixture of two or more thereof.

Optionally, the molar ratio of the compound of Formula (V) to the base in the Method 3 is 1:1-3, preferably 1:2.

Optionally, the reaction temperature of Method 3 is 0-60 °C, preferably 20-40 °C, more preferably 26-32 °C.

Optionally, the reaction time of Method 3 is 1-10 h, preferably 4-6 h.

More preferably, in Method 2 described above:
R¹⁰ is selected from methyl or deuterated methyl;
and/or R³, R⁴, R⁵, and R⁶ are independently selected from hydrogen, fluoro, chloro, methoxy, ethoxy or trifluoromethyl.

Moreover, Step 1 of Method 2 described above comprises the following reaction conditions:
The reaction conditions comprise a base; the base is selected from organic or inorganic bases. Preferably, the base includes any one of tetramethylguanidine, triethylamine, DBU, DIPEA, pyridine, sodium carbonate, potassium carbonate, cesium carbonate, potassium bicarbonate, sodium bicarbonate, lithium hydroxide, sodium hydroxide, and potassium hydroxide, or a mixture of two or more thereof. More preferably, the base includes any one of tetramethylguanidine, triethylamine, DBU, DIPEA, potassium carbonate, and cesium carbonate, or a mixture of two or more.

The reaction solvent of reaction condition is selected from organic solvents; preferably, the organic solvents comprise any one of isopropanol, ethanol, acetone, DMF, tetrahydrofuran, 2-methyltetrahydrofuran, and dioxane, or a mixture of two or more thereof.

Optionally, the molar ratio of the compound of Formula (IV) to the base in Step 1 is 1:1-3, preferably 1:2;
Optionally, the reaction temperature of Step 1 is 0-60 °C, preferably 20-40 °C, more preferably 26-32 °C.

Optionally, the reaction time in Step 1 is 1-10 h, preferably 4-6 h.
Moreover, Step 2 of Method 2 described above comprises the following reaction conditions:
The reaction conditions include an acid, the acid is selected from organic or inorganic acids. Preferably, the acid includes any one of hydrochloric acid, trifluoroacetic acid, sulfuric acid, phosphoric acid, acetic acid, and hydrobromic acid, or a mixture of two or more thereof. More preferably, the acid includes any one of hydrochloric acid, trifluoroacetic acid, and sulfuric acid, or a mixture of two or more thereof.

The reaction solvent of reaction condition is selected from organic solvents; preferably, the organic solvents comprise any one of isotetrahydrofuran, 2-methyltetrahydrofuran, dioxane, acetone, methanol, ethanol, isopropanol, and DMF, or a mixture of two or more thereof.
Optionally, the molar ratio of the compound of Formula (VII) to the acid in Step 2 is 1:1-30, preferably 10:20;
Optionally, the reaction temperature of Step 2 is -20-40 °C, preferably -10-10 °C, more preferably -5-5 °C.

Optionally, the reaction time in the Step 2 is 1-8 h, preferably 2-4 h.

Moreover, Step 3 of Method 2 described above comprises the following reaction conditions:
The reaction conditions comprise a base; the base is selected from organic or inorganic bases. Preferably, the base includes any one of triethylamine, DBU, DIPEA, tetramethylguanidine, pyridine, sodium carbonate, potassium carbonate, cesium carbonate, potassium bicarbonate, sodium bicarbonate, lithium hydroxide, sodium hydroxide, and potassium hydroxide, or a mixture of two or more thereof. More preferably, the base includes any one of triethylamine, DBU, DIPEA, tetramethylguanidine, potassium carbonate, and cesium carbonate, or a mixture of two or more thereof.

The reaction conditions include a condensing agent and a ligand. Preferably, the condensing agent and ligand include any one of EDCI, HOBT, HATU, HBTU, DCC, CDI, T3P, DPP-Cl, HCTU, TBTU, and DMAP or a mixture of two or more thereof. More preferably, the base includes any one of EDCI, HOBT, HATU, and HBTU or a mixture of two or more thereof.

The reaction solvent of reaction condition is selected from organic solvents; preferably, the organic solvents comprise any one of isotetrahydrofuran, DCM, 2-methyltetrahydrofuran, dioxane, acetonitrile, acetone, ethanol, isopropanol, DMF, and DMAC, or a mixture of two or more thereof.

Optionally, the molar ratio of the compound of Formula (VIII) to the base in the Step 3 is 1:1-5, preferably 1:3.

Optionally, the reaction temperature of Step 3 is 0-60 °C, preferably 20-40 °C, more preferably 26-32 °C.

Optionally, the reaction time in the Step 3 is 1-10 h, preferably 4-6 h,

Furthermore, the oxopyridine compounds of Formula (I) obtained via any of Methods 1, 2, or 3 possess at least one of the following features: (1) an enantiomeric excess (ee) for enantioselectivity of 98% or more, preferably 99% or more; (2) an N/O-alkylation selectivity ratio reaches 30-40:1 or more; (3) a yield of single step is 85% or more, preferably 90% or more; (4) a short production cycle with no complex post-treatment; (5) product purity of 98% or more.

Further, the oxopyridinone compounds of Formula (I) obtained via Methods 1, 2, and 3 may be subjected to crystallization, such as by applying the crystallization protocol disclosed in CN111770917A, to afford high-quality crystals, even with crystal quality that may exceed that reported in the publication.

Explanation of Terms:
"Alkyl" refers to lower alkyl, specifically C1 to C16 saturated branched or straight-chain alkyl. The alkyl portion of "alkylcarbonyl" is construed in the same way.
"Cycloalkyl" refers to C3 to C10 cycloalkyl, preferably C3to C6 cycloalkyl.
"Halogen" means F, Cl, Br, or I.
"Above" and "below" are inclusive.
DMAP: 4-dimethylaminopyridine
DEAD: diethyl azodicarboxylate
DIAD: diisopropyl azodicarboxylate
TMAD: azodicarboxamide
DTBAD: di-tert-butyl azodicarboxylate
ADDP: azodicarbonyldipiperidine
DBU: 1,8-diazabicyclo undec-7-ene
DIPEA: isopropyl ethylamine
T3P: 1-propyl phosphoric anhydride
DPP-Cl: diphenylphosphinic chloride
EDCI: 1-(3-dimethylaminopropyl)-3-ethylcarbodiimide hydrochloride
HOBT: 1-hydroxybenzotriazole
HBTU: benzotriazole-N,N,N',N'-tetramethylurea hexafluorophosphate
HATU: 2-(7-azobenzotriazole)-N,N,N',N'-tetramethyluronium hexafluorophosphate
DCC: N,N'-dicyclohexylcarbodiimide
DMF: N,N-dimethylformamide

Compared to the prior art, the present invention has the following advantages:
1. The novel preparation route of the present invention for the synthesis of the compound of formula (I) improves overall conversion and has absolute advantages in both enantioselectivity and N/O-alkylation selectivity. In particular, by using milder bases and more readily available solvents, a conversion wherein the N/O alkylation ratio is 30-40:1 or more can be achieved, with markedly fewer O-alkylated impurities. By contrast, in the prior art, the N-alkylation:O-alkylation ratio in the crude product is (9 to 10):1, with a higher level of O-alkylation impurities, and purification with respect to such isomeric impurities is extremely difficult. By comparison, the synthetic route according to the present invention possesses lower subsequent purification difficulty, which is beneficial for subsequent crystallization yield and improves the stability of crystallization refining process control.
2. Even more surprisingly, in the process for preparing the compound of formula (I) according to the present invention, the amorphous form of formula (I) with a substantially higher ee can be obtained. After extraction and solvent evaporation, the amorphous compound of formula (I) is obtained with an ee value of 98% or more, and even 99% or more. In the prior art, the ee value can be optimized only to 85 to 93%, with 7 to 15% isomeric impurities remaining. Accordingly, the crude obtained via the synthetic route of the present application, contains only 1-2% isomeric impurities, enabling direct isolation of crude product with >98% ee without a complicated chiral isomer purification process. The target crystal product can then be obtained through a subsequent crystallization Process, which greatly simplifies impurity removal, avoids complex post-treatment, reduces costs, and facilitates industrial-scale production.
3. The yield of the crude product prepared via single-step condensation reaction is increased to 85-98% or more, and overall route yield reaches ≥60-70% or more, compared with only 20-25% for prior art. Consequently, the synthetic route exhibits high conversion, greatly shortens the production cycle, avoids complicated post-treatment, reduces costs, and is well suited for industrial scale-up.

### DESCRIPTION OF THE EMBODIMENTS

The invention is further described in detail below with reference to the following examples and experimental examples. The examples and experiment examples are provided merely to illustrate the technical solution of the invention and are not intended to limit the invention. Any equivalent substitutions made by those skilled in the art in light of the present disclosure are deemed to fall within the scope of the present invention.

Compounds of the present invention, their stereoisomers, or the pharmaceutically acceptable salt may be prepared selecting the synthetic routes described in the Examples. The conventional reaction conditions for raw materials and solvents may be adjusted as required by substituents or salt formation. Such adjustments may be implemented by those skilled in the art based on the present disclosure. Unless otherwise specified, "column chromatography" of the present invention refers to silica gel column chromatography. Unless stated otherwise, the eluent solvent- whether a single solvent or a mixture- may be determined with reference to the reaction solvent and the common general knowledge or routine methods of those skilled in the art.

The structures of the compounds were determined by nuclear magnetic resonance (1H NMR) or liquid mass spectrometry (LC-MS).

The liquid chromatography-mass spectrometry (LC-MS) was an Agilent G6120B equipped with an Agilent 1260; the nuclear magnetic resonance (1H NMR) instrument was either a Bruker AVANCE-400 or a Bruker AVANCE-800; and the nuclear magnetic resonance (1H NMR) shifts (δ) were reported in parts per million (ppm), with DMSO as the measurement solvent and TMS as the internal standard. The chemical shifts were given in units of 10-6 (ppm).

The term "room temperature" according to the present invention refers to a temperature between 10 and 30°C.

Unless otherwise specified, the mixed-solvent ratios refer to volume ratios.

The term "20 ml ethyl acetate:n-heptane 1:2 solvent" in the present invention means 20 ml of a mixed solvent (ethyl acetate:n-heptane = 1:2, v/v). Similar expressions shall be construed in the same manner.

### Part 1. Examples 1-7

### Example 1: Preparation of (S)-2-fluoro-4-(2-(4-(5-chloro-2-(4-(trifluoromethyl)-1H-1,2,3-triazol-1-yl)phenyl)-5-methoxy-2 -oxopyridin-1(2H)-yl)butanamido)benzamide (Compound 1);

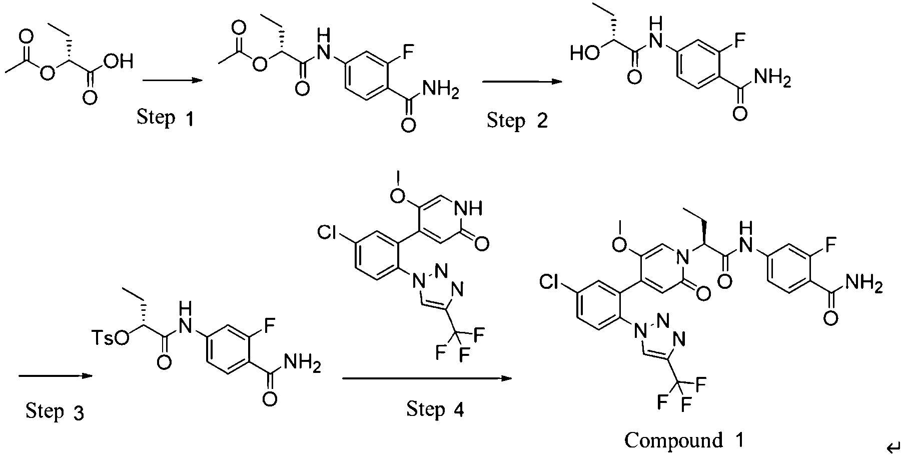

### Step 1: Preparation of (R)-1-((4-carbamoyl-3-fluorophenyl)amino)-1-oxobutan-2-yl acetate

1.03 g (7.05 mmol) of (R)-2-acetoxybutanoic acid was dissolved in 20 ml tetrahydrofuran. 720 mg (4.70 mmol) of 4-amino-2-chlorobenzamide was added, and the mixture was cooled to 0 °C or less. After the addition of pyridine (1.12 g, 14.1 mmol), 4.50g (14.1 mmol) of 1-propylphosphoric anhydride (50% solution in ethyl acetate) diluted with 10 ml tetrahydrofuran, was introduced dropwise. Upon completion of the addition, the reaction mixture was stirred at 0-5°C for 10 min, and then stirred at room temperature for 30 min. After completion of the reaction, the reaction was quenched with water and extracted with EA. The organic phase was washed sequentially with 5% citric acid, saturated sodium bicarbonate, water, and saturated brine; dried over anhydrous sodium sulfate, and the solvent was evaporated to afford the crude product. 10 ml of solvent of an ethyl acetate:n-heptane (1:1) was added to the crude product. The mixture was stirred at room temperature for 2 h and then filtered. The filter cake was washed with n-heptane, and dried in vacuo to give a white solid (86.1% yield, 99.56% ee, and 96.20% purity).

ESI-MS:m/z=283.1(M+H)⁺.

¹H NMR (400 MHz, DMSO-d6) δ: 10.76 (s, 1H), 8.08 - 7.77 (m, 1H), 7.92 - 7.88 (m, 1H), 7.76 (s, 2H), 7.60-7.56 (m, 1H), 4.56 (m, 1H), 2.25 (s, 3H), 1.98 - 1.88 (m, 2H), 0.88 (t, 3H).

Step 2. Preparation of (R)-2-fluoro-4-(2-hydroxybutanamido)benzamide.

1.5 g (5.32 mmol) of (R)-1-((4-carbamoyl-3-fluorophenyl)amino)-1-oxobutan-2-yl acetate was dissolved in a mixed solvent of methanol (10 ml) and water (15 ml). 2.20 g (15.96 mmol) of potassium carbonate was added, and the mixture was stirred at room temperature overnight. When the reaction was monitored by TLC and found completed, the reaction was quenched by slow addition of water (20 ml), and a large amount of white solid was precipitated. A further 50 ml of water was then added, and the resulting slurry was stirred for 1 h and then filtered. The filter cake was dried under vacuum to afford the product as a white solid in 87.3% yield, with 98.87% ee, and 96.82% purity.

ESI-MS:m/z=241.1(M+H)⁺.

¹H NMR (400 MHz, DMSO-d6) δ: 10.78 (s, 1H), 8.08 - 7.77 (m, 1H), 7.92 - 7.88 (m, 1H), 7.78 (s, 2H), 7.60-7.56 (m, 1H), 5.52 (s, 1H), 4.56 (m, 1H), 2.01 - 1.88 (m, 2H), 0.88 (t, 3H).

### Step 3: Preparation of (R)-1-((4-amino-3-fluorophenyl)amino)-1-oxobutan-2-yl p-toluenesulfonate

860 mg (3.58 mmol) of (R)-2-fluoro-4-(2-hydroxybutanamido)benzamide was dissolved in 10 ml dichloromethane. Then 723 mg (7.16 mmol) of triethylamine and 88 mg (0.72 mmol) of DMAP was added. Finally, a solution of p-toluenesulfonic acid (3.95 mmol) in dichloromethane (5 ml) was added dropwise at approximately 0°C, and the mixture was stirred at room temperature overnight. When the reaction was monitored by TLC and found completed, the reaction was quenched by slow addition of water (20 ml). The organic phase was obtained by extraction with dichloromethane, dried, and concentrated to obtain a crude product. The product was dissolved in 8 ml of ethyl acetate, and cooled to induce crystallization, affording a pale-yellow solid (86.6% yield; 98.52% ee; purity 95.65%).

ESI-MS:m/z=395.1(M+H)⁺.

¹H NMR (400 MHz, DMSO-d6) δ: 10.78 (s, 1H), 8.08-7.97 (m, 1H), 7.96 (s, 2H), 7.78-7.75 (m, 3H), 7.60-7.56 (m, 1H), 7.50-7.45 (m, 2H), 4.56-4.52 (m, 1H), 2.43 (s, 3H), 2.01-1.88 (m, 2H), 0.88 (t, 3H).

### Step 4: Preparation of Compound 1

200 mg (0.539 mmol) of 4-(5-chloro-2-(4-(trifluoromethyl)-1H-1,2,3-triazol-1-yl)phenyl)-5-methoxypyridin-2(1H)-one was added into a 25 ml single-neck flask, and dissolved in 5 ml of dioxane with stirring. 150 mg (1.09 mmol) of potassium carbonate and 62 mg (0.539 mmol) of tetramethylguanidine were added, and the mixture was stirred for 5 min. (R)-1-((4-amino-3-fluorophenyl)amino)-1-oxobutan-2-yl p-toluenesulfonate (0.648 mmol) was then introduced, after which the reaction mixture was warmed to 35 °C and stirred until completion was confirmed by TLC. The N/O-alkylation conversion ratio was 40:1. The reaction was quenched with the addition of saturated ammonium chloride, and extracted with ethyl acetate. The organic phase was washed sequentially with water, saturated brine, and anhydrous sodium sulfate, and the solvent was evaporated to afford the crude product with 95.8% yield, and 99.24% ee.

ESI-MS:m/z=593.2(M+H)⁺.

¹H NMR (400 MHz, DMSO-d6) δ: 10.78 (s, 1H), 9.14 (s, 1H), 7.88 - 7.77 (m, 3H), 7.72 - 7.61 (m, 2H), 7.55 (d, 2H), 7.37 (dd, 1H), 7.13 (s, 1H), 6.54 (s, 1H), 5.52 (dd, 1H), 3.25 (s, 3H), 2.18 - 2.00 (m, 2H), 0.78 (t, 3H).

A high-quality target compound was obtained from the crude product by using the crystallization method described in CN111770917A.

### Example 2: Preparation of Compound 2

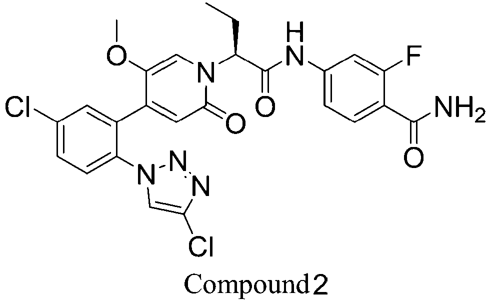

The preparation method was the same as that in Example 1. 4-(5-chloro-2-(4-(trifluoromethyl)-1H-1,2,3-triazol-1-yl)phenyl)-5-methoxypyridin-2(1H)-one in Step 4 was replaced with 4-(5-chloro-2-(4-chloro-1H-1,2,3-triazol-1-yl)phenyl)-5-methoxypyridin-2(1H)-one, thereby obtaining the crude title compound 2 in 90% yield and 98.61% ee. At the reaction endpoint, the N/O-alkylation conversion ratio was 35:1.

ESI-MS:m/z=559.1(M+H)⁺.

¹H NMR (400 MHz, DMSO-d6) δ:10.69 (s, 1H), 9.15 (d, 1H), 7.98 - 7.72 (m, 5H), 7.59 - 7.28 (m, 3H), 7.15 (s, 1H), 6.52 (s, 1H), 5.52 (dd 1H), 3.28 (s, 3H), 2.15-2.03 (m, 2H), 0.78 (t, 3H).

The crude product may yield a high-quality target compound using the crystallization method described in CN111770917A.

### Example 3: Preparation of Compound 3

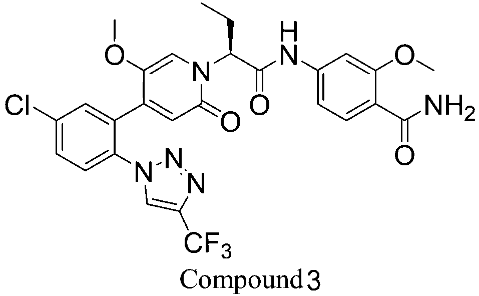

The preparation method was the same as in Example 1, except that 4-amino-2-fluorobenzamide in Step 1 was replaced with 4-amino-2-methoxy-benzamide , thereby obtaining the crude title compound 3 in 90% yield and 98.39% ee. At the reaction endpoint, the N/O-alkylation ratio was 30:1.

ESI-MS:m/z=605.1(M+H)⁺.

¹H NMR (400 MHz, DMSO-d6) δ:10.68 (s, 1H), 9.14 (d, 1H), 7.95 - 7.73 (m, 4H), 7.56 (m, 2H), 7.46 (d, 1H), 7.19 (dd, 1H), 7.14 (s, 1H), 6.53 (s, 1H), 5.54 (dd, 1H), 3.86 (s, 3H), 3.25 (s, 3H), 2.16-2.04 (m, 2H), 0.78 (t, 3H).

### Example 4: Preparation of Compound 4

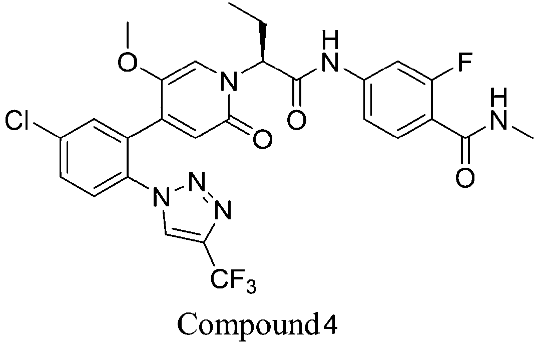

The preparation method was the same as in Example 1, except that 4-amino-2-fluorobenzamide in step 1 was replaced with 4-amino-2-fluoro-N-methylbenzamide, thereby obtaining the crude title compound 4 in 90% yield and 99.02% ee. At the reaction endpoint, the N/O-alkylation ratio was 40:1.

ESI-MS:m/z=623.1(M+H)⁺.

¹H NMR (400 MHz, DMSO-d6) δ: 10.82 (s, 1H), 9.16 (d, J = 1.1 Hz, 1H), 8.13 - 8.06 (m, 1H), 7.89 - 7.80 (m, 2H), 7.79 (d, J = 2.0 Hz, 1H), 7.70 - 7.60 (m, 2H), 7.37 (dd, J = 8.5, 2.0 Hz, 1H), 7.13 (s, 1H), 6.54 (s, 1H), 5.52 (t, J = 7.8 Hz, 1H), 3.25 (s, 3H), 2.76 (d, J = 4.6 Hz, 3H), 2.18 - 2.02 (m, 2H), 0.78 (t, J = 7.2 Hz, 3H).

### Example 5: Preparation of Compound 5

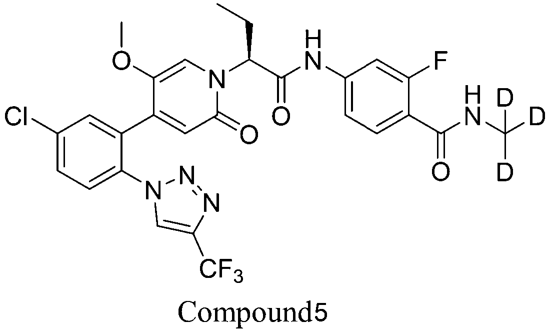

The preparation method was the same as in Example 1, except that 4-amino-2-fluorobenzamide in step 1 was replaced with 4-amino-2-fluoro-N-(methyl-d3)benzamide, thereby obtaining the crude title compound 5 in 89% yield and 98.87% ee. At the reaction endpoint, the N/O-alkylation ratio was 40:1.

ESI-MS:m/z=610.2(M+H)⁺.

¹H NMR (400 MHz, DMSO-d6) δ: 10.79 (s, 1H), 9.14 (d, J = 1.1 Hz, 1H), 8.06 (d, J = 3.4 Hz, 1H), 7.92 - 7.81 (m, 2H), 7.81 - 7.76 (m, 1H), 7.70 - 7.60 (m, 2H), 7.37 (dd, J = 8.6, 2.0 Hz, 1H), 7.13 (s, 1H), 6.54 (s, 1H), 5.51 (d, J = 8.6 Hz, 1H), 3.25 (s, 3H), 2.19 - 1.99 (m, J = 7.1 Hz, 2H), 0.78 (t, J = 7.2 Hz, 3H).

### Example 6: Preparation of Compound 6

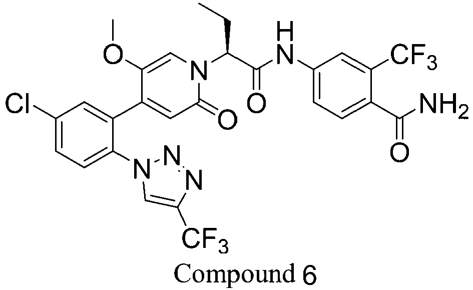

The preparation method was the same as in Example 1, except that 4-amino-2-fluorobenzamide in step 1 was replaced with 4-amino-2-trifluoromethyl-benzamide, thereby obtaining the crude title compound 6 in 89% yield and 98.87% ee. At the reaction endpoint, the N/O-alkylation ratio was 30:1.

ESI-MS:m/z=643.1(M+H)⁺.

¹H NMR (400 MHz, DMSO-d6) δ:10.80 (s, 1H), 9.13 (s, 1H), 8.12 (d, 1H), 8.02 - 7.72 (m, 5H), 7.54-7.51 (m, 2H), 7.14 (s, 1H), 6.54 (s, 1H), 5.51 (dd, 1H), 3.25 (s, 3H), 2.23 - 2.03 (m, 2H), 0.79 (t, 3H).

### Example 7: Preparation of Compound 7

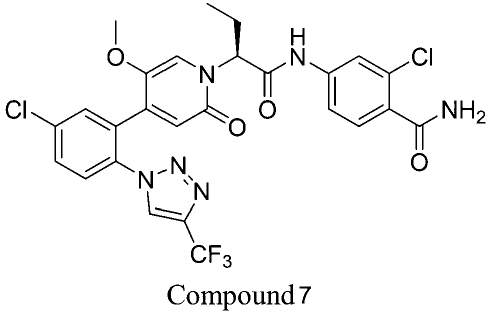

The preparation method was the same as in Example 1, except that 4-amino-2-fluorobenzamide in Step 1 was replaced with 4-amino-2-chloro-benzamide, thereby obtaining the crude title compound 7 in 92% yield and 98.87% ee. At the reaction endpoint, the N/O-alkylation ratio was 35:1.

ESI-MS:m/z=623.1(M+H)⁺.

¹H NMR (400 MHz, DMSO-d6) δ:10.58 (s, 1H), 9.11 (s, 1H), 7.91 - 7.66 (m, 4H), 7.58 - 7.25 (m, 3H), 7.15 (s, 1H), 6.51 (s, 1H), 5.53 (dd 1H), 3.26 (s, 3H), 2.88 (d, 3H),2.12-2.01 (m, 2H), 0.79 (t, 3H).

Table 1 lists the intermediates from the examples described above together with their NMR and MS data.

**Table 1**

| Structure & ID | Name | ¹H NMR | ESI-MS:m/z (M+H)⁺ |
|---|---|---|---|
| 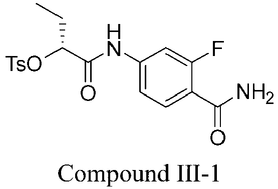 | (R)-1-((4-amino-3-fluorophenyl)a mino)-1-oxobuta n-2-yl p-toluenesulfonat e | ¹H NMR (400 MHz, DMSO-d6) δ: 10.78 (s, 1H), 8.08-7.97 (m, 1H), 7.96 (s, 2H), 7.78-7.75 (m, 3H), 7.60-7.56 (m, 1H), 7.50-7.45 (m, 2H), 4.56-4.52 (m, 1H), 2.43 (s, 3H), 2.01-1.88 (m, 2H), 0.88 (t, 3H). | 395.1 |
| 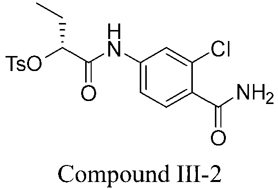 | (R)-1-((4-amino-3-chlorophenyl)a mino)-1-oxobuta n-2-yl p-toluenesulfonat e | ¹H NMR (400 MHz, DMSO-d6) δ: 10.78 (s, 1H), 8.18-8.07 (m, 1H), 8.01 (s, 2H), 7.89-7.81 (m, 3H), 7.62-7.58 (m, 1H), 7.50-7.45 (m, 2H), 4.56-4.53 (m, 1H), 2.43 (s, 3H), 2.01-1.88 (m, 2H), 0.88 (t, 3H). | 411.1 |
| 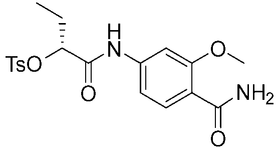 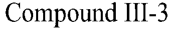 | (R)-1-((4-amino-3-methoxyphenyl )amino)-1-oxobut an-2-yl p-toluenesulfonat e | ¹H NMR (400 MHz, DMSO-d6) δ: 10.78 (s, 1H), 8.02-7.95 (m, 1H), 7.92 (s, 2H), 7.58-7.55 (m, 3H), 7.50-7.41 (m, 3H), 4.56-4.52 (m, 1H), 3.88 (s, 3H), 2.43 (s, 3H), 2.01-1.88 (m, 2H), 0.88 (t, 3H). | 407.1 |
| 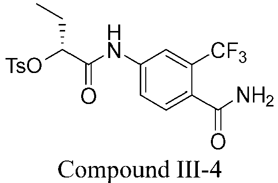 | (R)-1-((4-amino-3-trifluoromethyl phenyl)amino)-1-oxobutan-2-yl p-toluenesulfonat e | ¹H NMR (400 MHz, DMSO-d6) δ: 10.78 (s, 1H), 8.28-8.17 (m, 1H), 8.03 (s, 1H), 7.98-7.91 (m, 3H), 7.62-7.58 (m, 1H), 7.50-7.45 (m, 2H), 4.56-4.53 (m, 1H), 2.43 (s, 3H), 2.01-1.88 (m, 2H), 0.88 (t, 3H). | 445.02 |
| 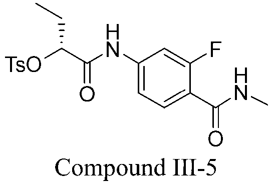 | (R)-1-((3-fluoro-4-(methylcarbam oyl)phenyl)amino )-1-oxobutan-2-yl p-toluenesulfonat e | ¹H NMR (400 MHz, DMSO-d6) δ: 10.78 (s, 1H), 8.12(s,1H), 8.08-7.97(m, 1H), 7.88-7.75(m, 3H), 7.60-7.56(m, 1H), 7.50-7.45(m, 2H), 4.56-4.52(m,1H), 2.83(s, 3H), 2.43 (s, 3H), 2.01-1.88 (m, 2H), 0.88 (t, 3H). | 409.1 |
| 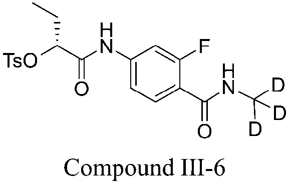 | (R)-1-((3-fluoro-4-((methyl-d3)car bamoyl)phenyl)a mino)-1-oxobuta n-2-yl p-toluenesulfonat e | ¹H NMR (400 MHz, DMSO-d6) δ: 10.78 (s, 1H), 8.12 (s, 1H), 8.08-7.97 (m, 1H), 7.88-7.75 (m, 3H), 7.60-7.56 (m, 1H), 7.50-7.45 (m, 2H), 4.56-4.52 (m, 1H), 2.43 (s, 3H), 2.01- 1.88 (m, 2H), 0.88 (t, 3H). | 412.1 |

The intermediates described above were prepared according to the same strategy as in Example 1, as set forth in Examples 1-7; namely, they correspond to the products obtained in Step 3 of the respective synthetic routes of Examples 1-7. The preparation methods for each intermediate are summarized below:

The compound of Formula (III-a) is reacted with p-toluenesulfonyl chloride under basic conditions to give: 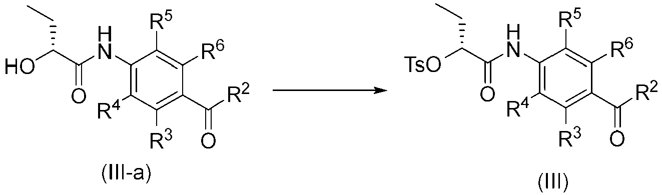 wherein, the definitions of R², R³, R⁴, R⁵, and R⁶ are defined the same as in any of the foregoing definitions provided herein.

It will be appreciated that the compound of formula (III-a-3) employed in Examples 1-7 is readily obtainable by those skilled in the art using common technical means; accordingly, further details are omitted herein.

### Part 2. Examples 8-15

**Example 8:** Preparation of (S)-4-(2-(4-(5-chloro-2-(4-(trifluoromethyl)-1H-1,2,3-triazol-1-yl)phenyl)-5-methoxy-2-oxopyri din-1(2H)-yl)butamido)-2-fluoro-N-methylbenzamide (Compound 4)

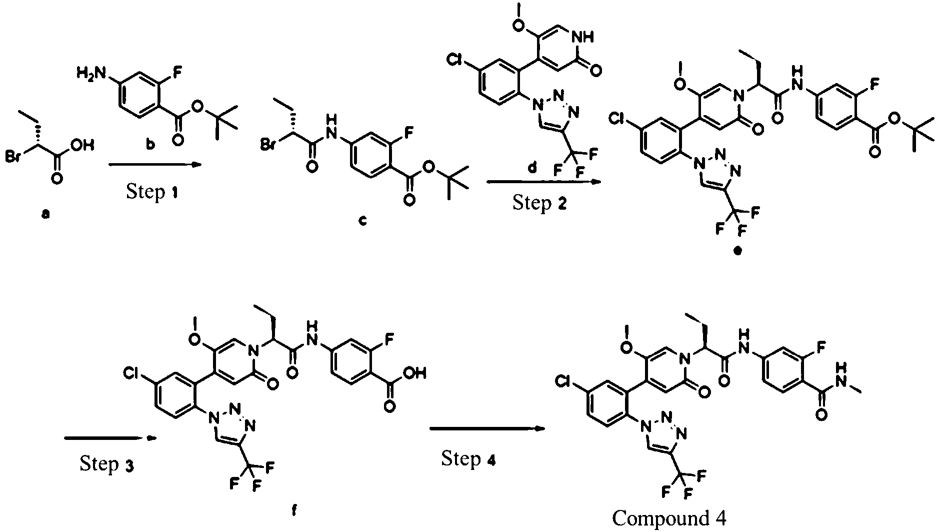

### Step 1. Preparation of tert-butyl (R)-4-(2-bromo butyramide) 2-fluoro-benzoate

441 mg (2.64 mmol) of (R)-2-bromo-3-propionic acid was dissolved in 4 ml tetrahydrofuran. 372 mg (1.76 mmol) of tert-butyl 4-amino-2-fluorobenzoate was added, and the mixture was cooled to 0 °C or less. After the addition of pyridine (654 mg, 8.27 mmol), 2.24 g (3.52 mmol) of 1-propylphosphoric anhydride (50% ethyl acetate solution) diluted with 2 ml tetrahydrofuran, was introduced dropwise. Upon completion of the addition, the reaction was stirred at 0-5°C for 10 min, and then stirred at room temperature for 30 min. After completion of the reaction, the reaction was quenched with water and extracted with EA. The organic phase was washed sequentially with 5% citric acid, saturated sodium bicarbonate, water, and saturated brine; dried over anhydrous sodium sulfate, and the solvent was evaporated to provide 608 mg of the crude product. 5 ml of an ethyl acetate was added to the crude product. The mixture was stirred at room temperature for 2 h and then filtered. The filter cake was washed with ethyl acetate, and dried in vacuo to give a white solid (74.0% yield, and 97.55% purity).

ESI-MS:m/z=360.1(M+H)⁺.

Step 2: Preparation of tert-butyl 2-fluoro-4-((S)-2-(4-(5-chloro-2-(4-(trifluoromethyl)-1H-1,2,3-triazol-1-yl)phenyl)-5-methoxy-2 -oxopyridin-1(2H)-yl)butanamido)benzoate.

2.0 g (5.39 mmol) of 4-(5-chloro-2-(4-(trifluoromethyl)-1H-1,2,3-triazol-1-yl)phenyl)-5-methoxypyridin-2(1H)-one was mixed with 50 ml isopropanol, and 20 ml of acetone. 1.87mg (16.24mmol) of tetramethylguanidine was added and the mixture was stirred for 5 min. 2.33 mg (6.48 mmol) of tert-butyl -(R)4-(2- butyramide) 2-fluoro benzoate was then added, and the mixture was stirred at room temperature overnight. After completion of the reaction, the reaction was quenched with saturated ammonium chloride t and extracted with ethyl acetate. The organic phase was washed sequentially with water, saturated brine and dried over anhydrous sodium sulfate. The solvent was evaporated to provide 4.60 mg of the crude product. After column chromatography (eluded with ethyl acetate:petroleum ether = 1:2), the product was collected as a white solid (2.58 g). 73.7% yield, 97.82% purity.

ESI-MS:m/z=650.2(M+H)⁺.

Step 3 : Preparation of (S)-4-(2-(4-(5-chloro-2-(4-(trifluoromethyl)-1H-1,2,3-triazol-1-yl)phenyl)-5-methoxy-2-oxopyri din-1(2H)-yl)butamido)-2-fluorobenzoic acid

2.58 g (3.97 mmol) of tert-butyl 2-fluoro-4-((S)-2-(4-(5-chloro-2-(4-(trifluoromethyl)-1H-1,2,3-triazol-1-yl)phenyl)-5-methoxy-2 -oxopyridin-1(2H)-yl)butanamido)benzoate was dissolved in 25 ml of acetonitrile. The internal temperature was lowered to 0 °C while monitoring, and 25 ml of concentrated hydrochloric acid was slowly added dropwise. TLC monitoring indicated complete reaction after 0.5 h. The reaction was quenched with water and extracted with EA. The organic phase was washed sequentially with saturated sodium bicarbonate, water, and saturated brine; dried over anhydrous sodium sulfate, and the solvent was evaporated to provide 2.36 g of the crude product. 15 ml of ethyl acetate was added to the crude product and the mixture was stirred to be dissolved. The insoluble solids were removed by filtration. The filtrate (mother liquor) was concentrated, and then stirred in 20 ml of ethyl acetate:n-heptane (1:2) solvent at room temperature for 2 h and then filtered. The filter cake was washed with n-heptane, and dried in vacuo to give 2.06 g of white solid (87.3% yield, 99.36% ee, 98.20% purity).

ESI-MS:m/z=594.1(M+H)⁺.

Step 4: Preparation of (S)-4-(2-(4-(5-chloro-2-(4-(trifluoromethyl)-1H-1,2,3-triazol-1-yl)phenyl)-5-methoxy-2-oxopyri din-1(2H)-yl)butamido)-2-fluoro-N-methylbenzamide

1.0 g (1.675 mmol) of (S)-4-(2-(4-(5-chloro-2-(4-(trifluoromethyl)-1H-1,2,3-triazol-1-yl)phenyl)-5-methoxy-2-oxopyri din-1(2H)-yl)butamido)-2-fluorobenzoic acid was dissolved in 10 ml DMAC. 226 mg (3.35 mmol) of methylamine hydrochloride and 953 mg (2.52 mmol) of HBTU were added. The internal temperature was lowered to 5 °C while monitored, and 1.1 g (8.38 mmol) of DIPEA was added dropwise. After maintaining the reaction at 5-10 °C for 1 h, the completion of reaction was indicated by TLC. The reaction was quenched by slow addition of 30 ml of water, and a gray-white precipitate gradually formed. The slurry was stirred for 1 h, the solids were collected by filtration, and was air-dried to provide 1.06 g of the crude product. Then, 20 ml of an ethyl acetate:n-heptane (1:2) solvent was added to the crude product. The mixture was stirred at room temperature for 2 h and then filtered. The filter cake was washed with n-heptane, and dried in vacuo to give 860 mg of white solid (84.3% yield, 99.27% ee, and 98.32% purity).

ESI-MS:m/z=607.2(M+H)⁺.

¹H NMR (400 MHz, DMSO-d6) δ: 10.82 (s, 1H), 9.16 (d, J = 1.1 Hz, 1H), 8.13 - 8.06 (m, 1H), 7.89 - 7.80 (m, 2H), 7.79 (d, J = 2.0 Hz, 1H), 7.70 - 7.60 (m, 2H), 7.37 (dd, J = 8.5, 2.0 Hz, 1H), 7.13 (s, 1H), 6.54 (s, 1H), 5.52 (t, J = 7.8 Hz, 1H), 3.25 (s, 3H), 2.76 (d, J = 4.6 Hz, 3H), 2.18 - 2.02 (m, 2H), 0.78 (t, J = 7.2 Hz, 3H).

### Example 9: Preparation of (S)-4-(2-(4-(5-chloro-2-(4-(trifluoromethyl)-1H-1,2,3-triazol-1-yl)phenyl)-5-methoxy-2-oxopyri din-1(2H)-yl)butamido)-2-fluoro-N-(methyl-d3) benzamide (Compound 5)

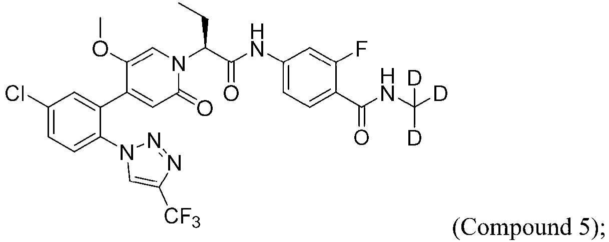

The preparation method was the same as in Example 8, except that the methylamine hydrochloride in Step 4 was replaced with deuterated methylamine hydrochloride, thereby obtaining the crude title compound 5 in 87% yield, 98.02% ee, and 98.02% purity.

ESI-MS:m/z=610.2(M+H)⁺.

¹H NMR (400 MHz, DMSO-d6) δ: 10.79 (s, 1H), 9.14 (d, J = 1.1 Hz, 1H), 8.06 (d, J = 3.4 Hz, 1H), 7.92 - 7.81 (m, 2H), 7.81 - 7.76 (m, 1H), 7.70 - 7.60 (m, 2H), 7.37 (dd, J = 8.6, 2.0 Hz, 1H), 7.13 (s, 1H), 6.54 (s, 1H), 5.51 (d, J = 8.6 Hz, 1H), 3.25 (s, 3H), 2.19 - 1.99 (m, J = 7.1 Hz, 2H), 0.78 (t, J = 7.2 Hz, 3H).

### Example 10: Preparation of Compound 8

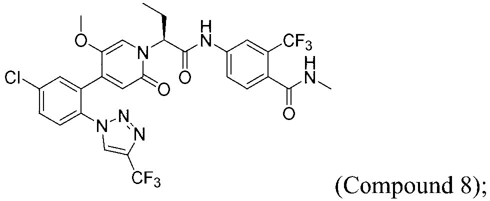

The preparation method was the same as in Example 8, except that tert-butyl 4-amino-2-fluorobenzoate in Step 1 was replaced with tert-butyl 4-amino-2-(trifluoromethyl)benzoate, thereby obtaining the title compound 8 with 99.17% ee and 97.62% purity.

ESI-MS:m/z=657.2(M+H)⁺.

¹H NMR (400 MHz, DMSO-d6) δ: 10.82 (s, 1H), 9.16 (d, J = 1.1 Hz, 1H), 8.18 - 8.12 (m, 1H), 7.98 - 7.90 (m, 2H), 7.85 (d, J = 2.0 Hz, 1H), 7.70 - 7.60 (m, 2H), 7.37 (dd, J = 8.5, 2.0 Hz, 1H), 7.13 (s, 1H), 6.54 (s, 1H), 5.53 (t, J = 7.8 Hz, 1H), 3.26 (s, 3H), 2.77 (d, J = 4.6 Hz, 3H), 2.18 - 2.02 (m, 2H), 0.78 (t, J = 7.2 Hz, 3H).

### Example 11: Preparation of Compound 9

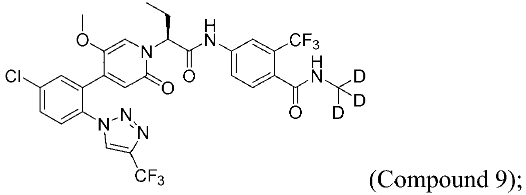

The preparation method was the same as in Example 8, except that tert-butyl 4-amino-2-fluorobenzoate in Step 1 was replaced with tert-butyl 4-amino-2-(trifluoromethyl)benzoate, the methylamine hydrochloride in Step 4 was replaced with deuterated methylamine hydrochloride, thereby obtaining the title compound 9 with 98.92% ee and 98.50% purity.

ESI-MS:m/z=660.2(M+H)⁺.

¹H NMR (400 MHz, DMSO-d6) δ: 10.82 (s, 1H), 9.15 (d, J = 1.1 Hz, 1H), 8.19 - 8.12 (m, 1H), 7.98 - 7.91 (m, 2H), 7.85 (d, J = 2.0 Hz, 1H), 7.70 - 7.60 (m, 2H), 7.37 (dd, J = 8.5, 2.0 Hz, 1H), 7.13 (s, 1H), 6.54 (s, 1H), 5.53 (t, J = 7.8 Hz, 1H), 3.26 (s, 3H), 2.18 - 2.02 (m, 2H), 0.78 (t, J = 7.2 Hz, 3H).

### Example 12: Preparation of Compound 10

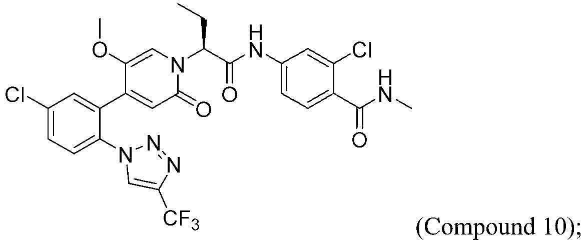

The preparation method was the same as in Example 8 except that tert-butyl 4-amino-2-fluorobenzoate in Step 1 was replaced with tert-butyl 4-amino-2-chlorobenzoate, thereby obtaining the title compound 10 with 98.89% ee and 98.49% purity.

ESI-MS:m/z=623.1(M+H)⁺.

¹H NMR (400 MHz, DMSO-d6) δ: 10.82 (s, 1H), 9.16 (d, J = 1.1 Hz, 1H), 8.16 - 8.10 (m, 1H), 7.96 - 7.88 (m, 2H), 7.84 (d, J = 2.0 Hz, 1H), 7.70 - 7.60 (m, 2H), 7.37 (dd, J = 8.5, 2.0 Hz, 1H), 7.13 (s, 1H), 6.54 (s, 1H), 5.52 (t, J = 7.8 Hz, 1H), 3.26 (s, 3H), 2.76 (d, J = 4.6 Hz, 3H), 2.18 - 2.02 (m, 2H), 0.78 (t, J = 7.2 Hz, 3H).

### Example 13: Preparation of Compound 11

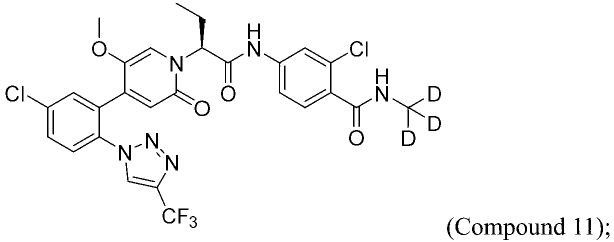

The preparation method was the same as in Example 8 except that tert-butyl 4-amino-2-fluorobenzoate in Step 1 was replaced with tert-butyl 4-amino-2-chlorobenzoate, the methylamine hydrochloride in Step 4 was replaced with deuterated methylamine hydrochloride, thereby obtaining the title compound 11 with 98.96% ee and 98.32% purity.

ESI-MS:m/z=626.1(M+H)⁺.

¹H NMR (400 MHz, DMSO-d6) δ: 10.81 (s, 1H), 9.16 (d, J = 1.1 Hz, 1H), 8.16 - 8.10 (m, 1H), 7.96 - 7.89 (m, 2H), 7.84 (d, J = 2.0 Hz, 1H), 7.70 - 7.60 (m, 2H), 7.37 (dd, J = 8.5, 2.0 Hz, 1H), 7.13 (s, 1H), 6.54 (s, 1H), 5.52 (t, J = 7.8 Hz, 1H), 3.26 (s, 3H), 2.18 - 2.06 (m, 2H), 0.78 (t, J = 7.2 Hz, 3H).

### Example 14: Preparation of Compound 12

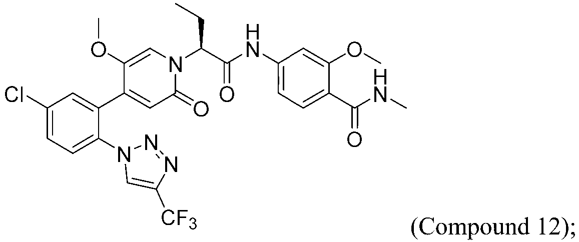

The preparation method was the same as in Example 8 except that tert-butyl 4-amino-2-fluorobenzoate in Step 1 was replaced with tert-butyl 4-amino-2-(methoxy)benzoate, thereby obtaining the title compound 12 with 98.68% ee and 98.65% purity.

ESI-MS:m/z=619.2(M+H)⁺.

¹H NMR (400 MHz, DMSO-d6) δ: 10.82 (s, 1H), 9.16 (d, J = 1.1 Hz, 1H), 8.13 - 8.06 (m, 1H), 7.89 - 7.80 (m, 2H), 7.79 (d, J = 2.0 Hz, 1H), 7.70 - 7.60 (m, 2H), 7.37 (dd, J = 8.5, 2.0 Hz, 1H), 7.13 (s, 1H), 6.54 (s, 1H), 5.52 (t, J = 7.8 Hz, 1H), 3.86 (s, 3H), 3.25 (s, 3H), 2.76 (d, J = 4.6 Hz, 3H), 2.18 - 2.02 (m, 2H), 0.78 (t, J = 7.2 Hz, 3H).

### Example 15: Preparation of Compound 13

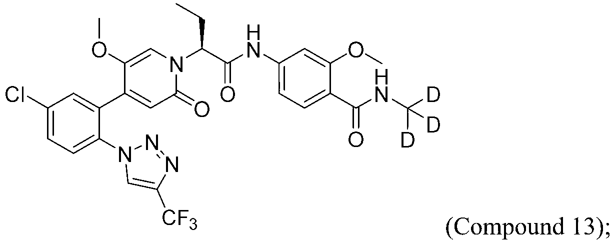

The preparation method was the same as in Example 8 except that tert-butyl 4-amino-2-fluorobenzoate in Step 1 was replaced with tert-butyl 4-amino-2-(methoxy)benzoate, the methylamine hydrochloride in Step 4 was replaced with deuterated methylamine hydrochloride, thereby obtaining the title compound 13 with 98.68% ee and 98.95% purity.

ESI-MS:m/z=622.2(M+H)⁺.

¹H NMR (400 MHz, DMSO-d6) δ: 10.81 (s, 1H), 9.17 (d, J = 1.1 Hz, 1H), 8.13 - 8.08 (m, 1H), 7.89 - 7.81 (m, 2H), 7.79 (d, J = 2.0 Hz, 1H), 7.70 - 7.60 (m, 2H), 7.37 (dd, J = 8.5, 2.0 Hz, 1H), 7.13 (s, 1H), 6.54 (s, 1H), 5.53 (t, J = 7.8 Hz, 1H), 3.86 (s, 3H), 3.25 (s, 3H), 2.18 - 2.02 (m, 2H), 0.78 (t, J = 7.2 Hz, 3H).

### Comparative Example 1: Preparation of (S)-4-(2-(4-(5-chloro-2-(4-(trifluoromethyl)-1H-1,2,3-triazol-1-yl)phenyl)-5-methoxy-2-oxopyri din-1(2H)-yl)butamido)-2-fluoro-N-methylbenzamide (Compound 4)

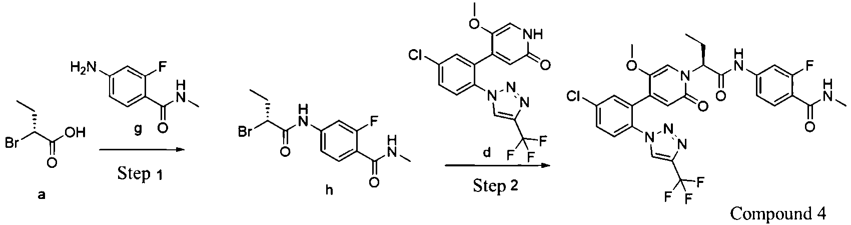

The title Compound 4 was synthesized following the synthetic strategy disclosed in CN 111770917 A, with 82.12% ee and 96.26% purity. Given that no enantiomeric co-crystal formed during crystallization of Compound 4, the chiral isomer of Compound 4 (R-configuration compound 4) could not be removed during purification.

However, the route provided by the present invention, such as, example 8, provided an intermediate of Formula (VIII) with an ee- value higher than 98% via chemical resolution of compound of Formula (VIII), thereby achieving the target API of high ee value and high chemical purity for compound 4.

The NMR and MS data for intermediates derived from Compound 8-14 above employing the procedures described in the Examples are shown in Table 2:

**Table 2**

| Structure | Name | ¹H NMR | ESI-MS:m/z (M+H)⁺ |
|---|---|---|---|
| 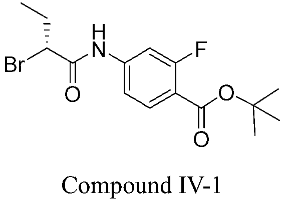 | tert-butyl -(R)-4-(2-bro mobutyramid e)amino 2-fluoro benzoate | ¹H NMR (400 MHz, DMSO-d6) δ: 10.78 (s, 1H), 8.08 - 7.97 (m, 1H), 7.92 - 7.88 (m, 1H), 7.60-7.56 (m, 1H), 4.86-4.83 (m, 1H), 2.18 - 2.02 (m, 2H), 1.45 (s, 9H), 0.87 (t, J = 7.2 Hz, 3H). | 360.1 |
| 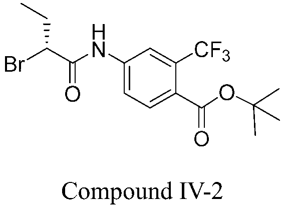 | tert-butyl (R) -4-(2-bromob utyramide)-2-(trifluorometh yl) benzoate | ¹H NMR (400 MHz, DMSO-d6) δ: 10.79 (s, 1H), 8.27 - 8.17 (m, 1H), 8.05 - 7.98 (m, 1H), 7.75-7.69 (m, 1H), 4.86-4.83 (m, 1H), 2.18 - 2.02 (m, 2H), 1.45 (s, 9H), 0.87 (t, J = 7.2 Hz, 3H). | 410.1 |
| 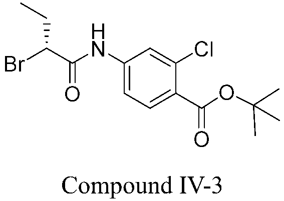 | tert-butyl (R) -4-(2-bromob utyramide)-2-chloro-benzo ate | ¹H NMR (400 MHz, DMSO-d6) δ: 10.79 (s, 1H), 8.18 - 8.07 (m, 1H), 7.95 - 7.90 (m, 1H), 7.65-7.59 (m, 1H), 4.86-4.83 (m, 1H), 2.18 - 2.02 (m, 2H), 1.45 (s, 9H), 0.87 (t, J = 7.2 Hz, 3H). | 378.0 |
| 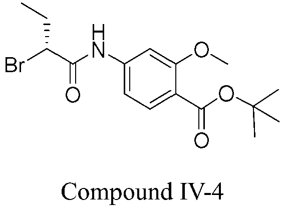 | tert-butyl - (R) -4-(2-bromob utyramide) -2-methoxy benzoate) | ¹H NMR (400 MHz, DMSO-d6) δ: 10.78 (s, 1H), 8.01 - 7.95 (m, 1H), 7.82 - 7.78 (m, 1H), 7.58-7.52 (m, 1H), 4.86-4.83 (m, 1H), 3.86 (s, 3H), 2.18 - 2.02 (m, 2H), 1.45 (s, 9H), 0.87 (t, J = 7.2 Hz, 3H). | 372.1 |

### Part 3. Examples 16-43

### Part 3. First Set of Examples

Examples 16-22 provide a class of intermediates and the oxopyridine compounds of formula (I) synthesized therefrom, in which R⁷ is a trifluoromethanesulfonyl group. The specific synthetic route was as follows:

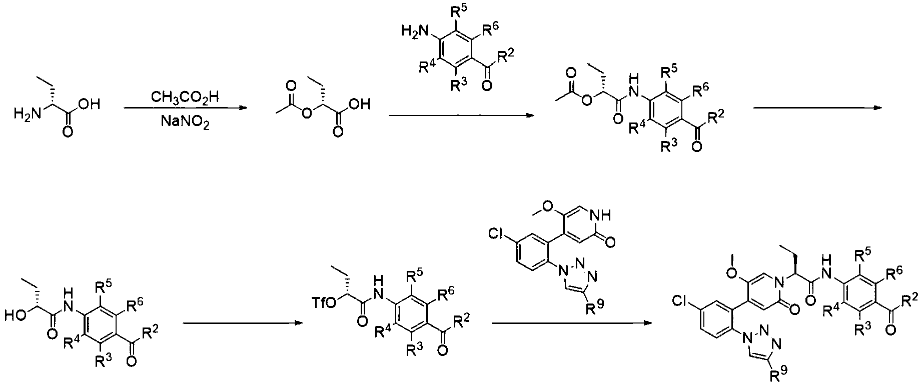

### Example 16: Preparation of Compound 1

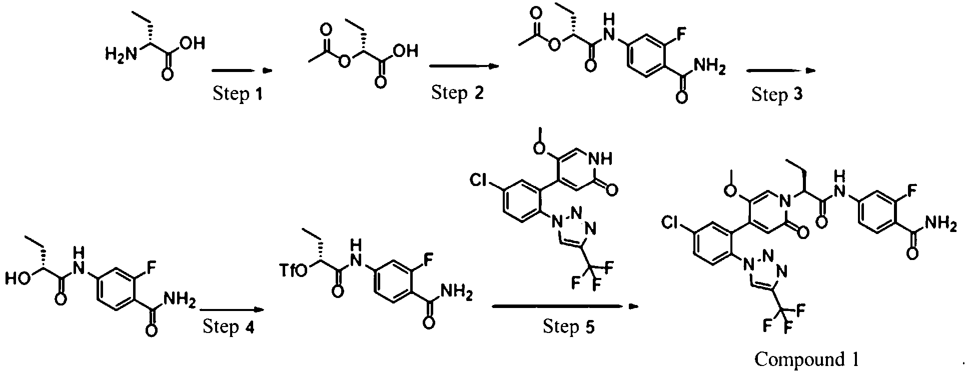

### Step 1: (R)-2-acetoxybutanoic acid

5.0 g (48.49 mmol) of D-2-aminobutyric acid was dissolved in 20 g (mmol) acetic acid, and the mixture was cooled to 5 °C or less. 6.7 g (97.09 mmol) of sodium nitrite was added in portions while maintaining the temperature at 0-5 °C or less, the addition was completed in approximately 30 min, and the reaction solution was then stirred at 0-5 °C for 6-10 h. Subsequently, 3 vol of water was added to the reaction solution, and then the reaction solution was extracted with ethyl acetate three times. The organic phases were combined and then washed with a large amount of water, followed by a sodium bicarbonate solution washing to completely remove acetic acid, washed with saturated brine, dried over anhydrous sodium sulfate, and the solvent was evaporated to give 7.5 g of an oil product, which was used directly in the next step without purification.

ESI-MS:m/z=147.1(M+H)⁺.

### Step 2: Preparation of (R)-1-((4-carbamoyl-3-fluorophenyl)amino)-1-oxobutan-2-yl acetate

1.03 g (7.05 mmol) of (R)-2-acetoxybutanoic acid was dissolved in 20 ml tetrahydrofuran. 720 mg (4.70 mmol) of 4-amino-2-fluorobenzamide was added, and the mixture was cooled to 0 °C or less. After the addition of pyridine (1.12 g, 14.1 mmol), 4.50g (14.1 mmol) of 1-propylphosphoric anhydride (50% solution in ethyl acetate), diluted with 10 ml tetrahydrofuran, was introduced dropwise. Upon completion of the addition, the reaction mixture was stirred at 0-5°C for 10 min, and then stirred at room temperature for 30 min. After completion of the reaction, the reaction was quenched with water and extracted with EA. The organic phase was washed sequentially with 5% citric acid, saturated sodium bicarbonate, water, and saturated brine; dried over anhydrous sodium sulfate, and the solvent was evaporated to provide the crude product. 10 ml of an organic solvent mixture (ethyl acetate and n-heptane mixed in a volume ratio of 1:1) was added to the crude product. The mixture was stirred at room temperature for 2 h and then filtered. The filter cake was washed with n-heptane, and dried in vacuo to give a white solid (76.1% yield, 99.56% ee, 96.20% purity).

ESI-MS:m/z=283.1(M+H)⁺.

Step 3. Preparation of (R)-2-fluoro-4-(2-hydroxybutanamido)benzamide.

1.5 g (5.32 mmol) of (R)-1-((4-carbamoyl-3-fluorophenyl)amino)-1-oxobutan-2-yl acetate was dissolved in a mixed solvent of methanol (10 ml) and water (15 ml). 2.20 g (15.96 mmol) of potassium carbonate was added, and the mixture was stirred at room temperature overnight. When the reaction was monitored by TLC and found completed, the reaction was quenched by slow addition of water (20 ml), and a large amount of white solid was precipitated in the system. A further 50 ml of water was then added, and the resulting slurry was stirred for 1 h and then filtered. The cake was dried under vacuum to provide the product as a white solid in 87.3% yield, with 98.87% ee, and 96.82% purity.

ESI-MS:m/z=241.1(M+H)⁺.

### Step 4: Preparation of (R)-1-((4-amino-3-fluorophenyl)amino)-1-oxobutan-2-yl trifluoromethanesulfonate

860 mg (3.58 mmol) of (R)-2-fluoro-4-(2-hydroxybutanamido)benzamide was dissolved in 10 ml dichloromethane. Then 723 mg (7.16 mmol) of triethylamine and 88 mg (0.72 mmol) of 4-dimethylaminopyridine (DMAP) was added. Finally, a solution of trifluoromethanesulfonic acid (678mg, 3.95 mmol) in dichloromethane (5 ml) was added dropwise at about 0 °C, and the mixture was stirred at room temperature overnight. When the reaction was monitored by TLC and found completed, the reaction was quenched by slow addition of water (20 ml). The organic phase was obtained by extraction with dichloromethane, dried, and concentrated to obtain a crude product. The product was dissolved in 8 ml of ethyl acetate, and cooled to induce crystallization, obtaining a pale-yellow solid (86.6% yield; 98.52% ee; purity 95.65%).

ESI-MS:m/z=373.1(M+H)⁺.

### Step 5: Preparation of Compound 1

200 mg (0.539 mmol) of 4-(5-chloro-2-(4-(trifluoromethyl)-1H-1,2,3-triazol-1-yl)phenyl)-5-methoxypyridin-2(1H)-one was added into a 25 ml single-neck flask, and dissolved in 5 ml of dioxane with stirring. 150 mg (1.08 mmol) of potassium carbonate and 62 mg (0.539 mmol) of tetramethylguanidine were added, and the mixture was stirred for 5 min. (R)-1-((4-amino-3-fluorophenyl)amino)-1-oxobutan-2-yl trifluoromethanesulfonate (0.648 mmol) was then introduced, after which the reaction mixture was warmed to 35 °C and maintained with stirring until completion of reaction was confirmed by TLC. The N/O-alkylation conversion ratio was 40:1. The reaction was quenched with the addition of saturated ammonium chloride, and extracted with ethyl acetate. The organic phase was washed sequentially with water, saturated brine, and anhydrous sodium sulfate, and the solvent was evaporated to provide the crude product with 95.8% yield, and 98.51% ee.

ESI-MS:m/z=593.2(M+H)⁺.

¹H NMR (400 MHz, DMSO-d6) δ: 10.78 (s, 1H), 9.14 (s, 1H), 7.88 - 7.77 (m, 3H), 7.72 - 7.61 (m, 2H), 7.55 (d, 2H), 7.37 (dd, 1H), 7.13 (s, 1H), 6.54 (s, 1H), 5.52 (dd, 1H), 3.25 (s, 3H), 2.18 - 2.00 (m, 2H), 0.78 (t, 3H).

Crystallization of the crude product yielded a high-quality target compound.

### Example 17: Preparation of Compound 2

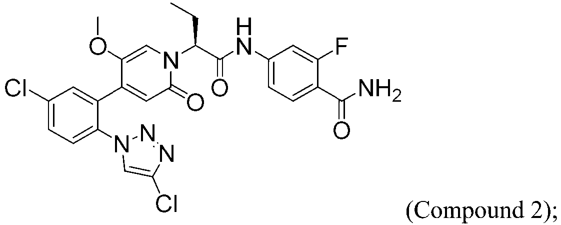

The preparation method was the same as that in Example 16, except that 4-(5-chloro-2-(4-(trifluoromethyl)-1H-1,2,3-triazol-1-yl)phenyl)-5-methoxypyridin-2(1H)-one in Step 5 was replaced with 4-(5-chloro-2-(4-chloro-1H-1,2,3-triazol-1-yl)phenyl)-5-methoxypyridin-2(1H)-one, i.e., R⁹ was converted from the trifluoromethyl group to a chloro group, thereby obtaining the crude compound 2 in 90% yield and 98.61% ee. At the reaction endpoint, the N/O-alkylation conversion ratio was 35:1.

ESI-MS:m/z=559.1(M+H)⁺.

¹H NMR (400 MHz, DMSO-d6) δ:10.69 (s, 1H), 9.15 (d, 1H), 7.98 - 7.72 (m, 5H), 7.59 - 7.28 (m, 3H), 7.15 (s, 1H), 6.52 (s, 1H), 5.52 (dd 1H), 3.28 (s, 3H), 2.15-2.03 (m, 2H), 0.78 (t, 3H).

Crystallization of the crude product yielded a high-quality target compound.

### Example 18: Preparation of Compound 3

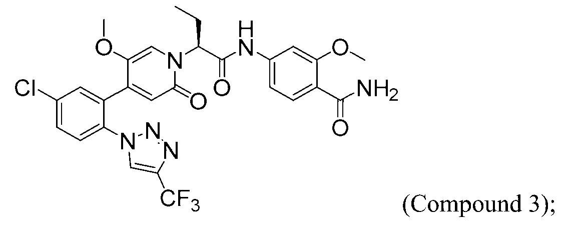

The preparation method was the same as in Example 1, except that 4-amino-2-fluorobenzamide in Step 2 was replaced with 4-amino-2-methoxybenzamide, i.e., R⁶ was converted from the fluoro group to a methoxy group, thereby obtaining crude compound 3 in 90% yield and 98.39% ee. At the reaction endpoint, the N/O-alkylation ratio was 30:1.

ESI-MS:m/z=605.1(M+H)⁺.

¹H NMR (400 MHz, DMSO-d6) δ:10.68 (s, 1H), 9.14 (d, 1H), 7.95 - 7.73 (m, 4H), 7.56 (m, 2H), 7.46 (d, 1H), 7.19 (dd, 1H), 7.14 (s, 1H), 6.53 (s, 1H), 5.54 (dd, 1H), 3.86 (s, 3H), 3.25 (s, 3H), 2.16-2.04 (m, 2H), 0.78 (t, 3H).

### Example 19: Preparation of Compound 4

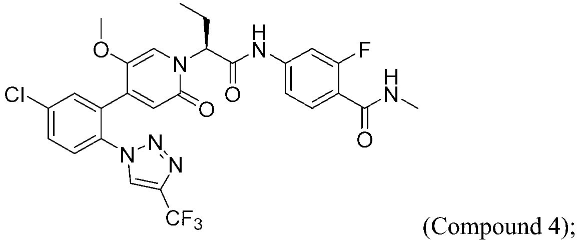

The preparation method was the same as in Example 16, except that 4-amino-2-fluorobenzamide in Step 2 was replaced with 4-amino-2-fluoro-N-methylbenzamide, i.e., the R² was converted from the amino group to a N-methyl group, thereby obtaining the crude compound 4 in 90% yield and 99.02% ee. At the reaction endpoint, the N/O-alkylation ratio was 40:1.

ESI-MS:m/z=623.1(M+H)⁺.

¹H NMR (400 MHz, DMSO-d6) δ: 10.82 (s, 1H), 9.16 (d, J = 1.1 Hz, 1H), 8.13 - 8.06 (m, 1H), 7.89 - 7.80 (m, 2H), 7.79 (d, J = 2.0 Hz, 1H), 7.70 - 7.60 (m, 2H), 7.37 (dd, J = 8.5, 2.0 Hz, 1H), 7.13 (s, 1H), 6.54 (s, 1H), 5.52 (t, J = 7.8 Hz, 1H), 3.25 (s, 3H), 2.76 (d, J = 4.6 Hz, 3H), 2.18 - 2.02 (m, 2H), 0.78 (t, J = 7.2 Hz, 3H).

### Example 20: Preparation of Compound 5

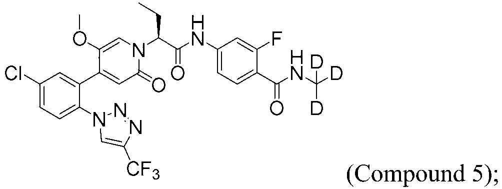

The preparation method was the same as in Example 16, except that 4-amino-2-fluorobenzamide in Step 2 was replaced with 4-amino-2-fluoro-N-(methyl-d3) benzamide, i.e., the R² was converted from the amino group to a N-(methyl-d3) group, thereby obtaining the crude compound 5 in 89% yield and 98.87% ee. At the reaction endpoint, the N/O-alkylation ratio was 40:1.

ESI-MS:m/z=610.2(M+H)⁺.

¹H NMR (400 MHz, DMSO-d6) δ: 10.79 (s, 1H), 9.14 (d, J = 1.1 Hz, 1H), 8.06 (d, J = 3.4 Hz, 1H), 7.92 - 7.81 (m, 2H), 7.81 - 7.76 (m, 1H), 7.70 - 7.60 (m, 2H), 7.37 (dd, J = 8.6, 2.0 Hz, 1H), 7.13 (s, 1H), 6.54 (s, 1H), 5.51 (d, J = 8.6 Hz, 1H), 3.25 (s, 3H), 2.19 - 1.99 (m, J = 7.1 Hz, 2H), 0.78 (t, J = 7.2 Hz, 3H).

### Example 21: Preparation of Compound 6

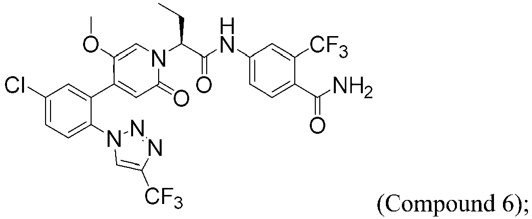

The preparation method was the same as in Example 16, except that 4-amino-2-fluorobenzamide in Step 2 was replaced with 4-amino-2-trifluoromethyl-N-methylbenzamide, i.e., R⁶ was converted from the fluoro group to a trifluoromethyl group, thereby obtaining crude compound 6 in 89% yield and 98.87% ee. At the reaction endpoint, the N/O-alkylation ratio was 30:1.

ESI-MS:m/z=643.1(M+H)⁺.

¹H NMR (400 MHz, DMSO-d6) δ:10.80 (s, 1H), 9.13 (s, 1H), 8.12 (d, 1H), 8.02 - 7.72 (m, 5H), 7.54-7.51 (m, 2H), 7.14 (s, 1H), 6.54 (s, 1H), 5.51 (dd, 1H), 3.25 (s, 3H), 2.23 - 2.03 (m, 2H), 0.79 (t, 3H).

### Example 22: Preparation of Compound 7

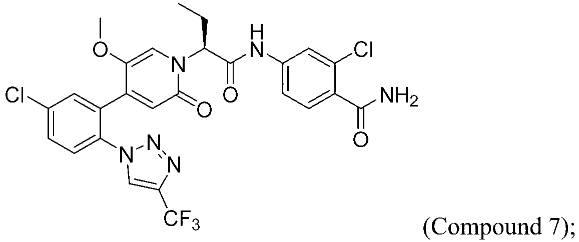

The preparation method was the same as in Example 16, except that 4-amino-2-fluorobenzamide in Step 2 was replaced with 4-amino-2-chlorobenzamide, i.e., R⁶ was converted from the fluoro group to a chloro group, thereby obtaining crude compound 7 in 92% yield and 98.87% ee. At the reaction endpoint, the N/O-alkylation ratio was 35:1.

ESI-MS:m/z=623.1(M+H)⁺.

¹H NMR (400 MHz, DMSO-d6) δ:10.58 (s, 1H), 9.11 (s, 1H), 7.91 - 7.66 (m, 4H), 7.58 - 7.25 (m, 3H), 7.15 (s, 1H), 6.51 (s, 1H), 5.53 (dd 1H), 3.26 (s, 3H), 2.88 (d, 3H),2.12-2.01 (m, 2H), 0.79 (t, 3H).

Table 3 lists the intermediates of the examples 16-22 together with their NMR and MS data.

**Table 3. Intermediates of the examples 16-22 together with their NMR and MS data.**

| Structure & ID | Name | ¹H NMR | ESI-MS:m/z (M+H)⁺ |
|---|---|---|---|
| 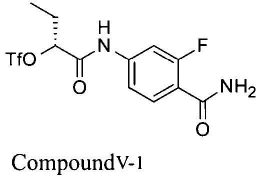 | (R)-1-((4-amino-3 -fluorophenyl)ami no)-1-oxobutan-2 -yl trifluoromethanes ulfonate | ¹H NMR (400 MHz, DMSO-d6) δ: 10.67 (s, 1H), 8.08 - 7.97 (m, 1H), 7.92 - 7.88 (m, 1H), 7.78 (s, 2H), 7.62-7.58 (m, 1H), 5.83 -5.76 (m, 1H), 1.96 - 1.88 (m, 2H), 0.88 (t, 3H). | 373.1 |
| 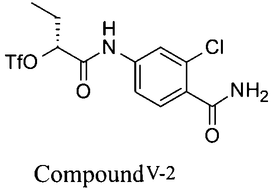 | (R)-1-((4-amino-3 - chlorophenyl)ami no)-1-oxobutan-2 -yl trifluoromethanes ulfonate | ¹H NMR (400 MHz, DMSO-d6) δ: 10.67 (s, 1H), 8.18-8.06 (m, 1H), 7.98-7.92 (m, 1H), 7.79 (s, 2H), 7.69-7.62 (m, 1H), 5.82 -5.76 (m, 1H), 1.96-1.88 (m, 2H), 0.86 (t, 3H). | 389.0 |
| 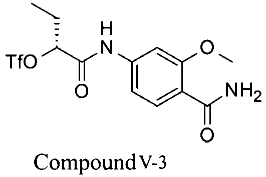 | (R)-1-((4-amino-3 -methoxyphenyl)a mino)-1-oxobutan -2-yl trifluoromethanes ulfonate | ¹H NMR (400 MHz, DMSO-d6) δ: 10.68 (s, 1H), 8.02-7.96 (m, 1H), 7.79 (s, 2H), 7.52-7.45 (m, 1H), 7.39-7.32 (m, 1H), 5.82 -5.76(m, 1H), 3.86 (s, 3H). 1.96-1.89(m, 2H), 0.86 (t, 3H). | 385.1 |
| 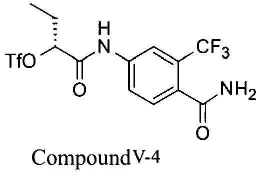 | (R)-1-((4-amino-3 -trifluoromethylp henyl)amino)-1-o xobutan-2-yl trifluoromethanes ulfonate | ¹H NMR (400 MHz, DMSO-d6) δ: 10.67 (s, 1H), 8.26-8.18 (m, 1H), 8.08-7.99 (m, 1H), 7.82 (s, 2H), 7.73-7.68 (m, 1H), 5.82-5.76 (m, 1H), 1.96-1.88 (m, 2H), 0.86 (t, 3H). | 423.1 |
| 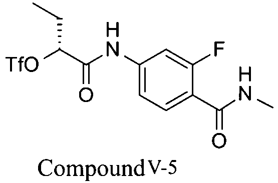 | (R)-1-((3-fluoro-4 -(methylcarbamo yl)phenyl)amino)-1-oxobutan-2-yl trifluoromethanes ulfonate | ¹H NMR (400 MHz, DMSO-d6) δ: 10.67 (s, 1H), 8.28 (s, 1H), 8.08 - 7.97 (m, 1H), 7.92 - 7.88 (m, 1H), 7.62-7.58 (m, 1H), 5.85 (m, 1H), 2.88 (s, 3H), 1.96 - 1.88 (m, 2H), 0.88 (t, 3H). | 387.1 |
| 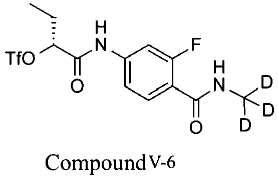 | (R)-1-((3-fluoro-4 -((methyl-d3)carb amoyl)phenyl)am ino)-1-oxobutan-2 -yl trifluoromethanes ulfonate | ¹H NMR (400 MHz, DMSO-d6) δ: 10.67 (s, 1H), 8.28 (s, 1H), 8.08 - 7.97 (m, 1H), 7.92 - 7.88 (m, 1H), 7.62-7.58 (m, 1H), 5.85 (m, 1H), 1.96 - 1.88 (m, 2H), 0.88 (t, 3H). | 390.1 |

### Part 3. Second Set of Examples

Examples 23-29 provide a class of intermediates and the oxopyridine compounds of formula (I) synthesized therefrom, in which R⁷ is a methanesulfonyl group. The details are as follows:

### Example 23: Preparation of (S)-2-fluoro-4-(2-(4-(5-chloro-2-(4-(trifluoromethyl)-1H-1,2,3-triazol-1-yl)phenyl)-5-methoxy-2 -oxopyridin-1(2H)-yl)butanamido)benzamide (Compound 1):

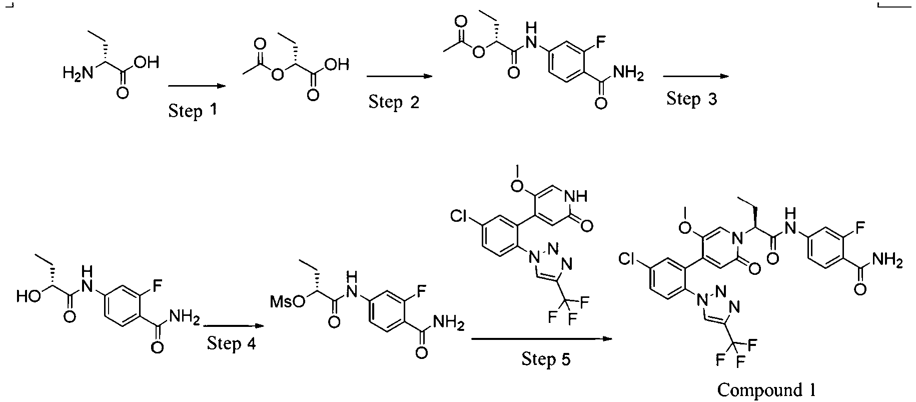

wherein, steps 1-3 and 5 of present Example are the same as those in Example 16, except that:
Step 4: Preparation of (R)-1-((4-amino-3-fluorophenyl)amino)-1-oxobutan-2-yl methanesulfonate

860 mg (3.58 mmol) of (R)-2-fluoro-4-(2-hydroxybutanamido)benzamide was dissolved in 10 ml dichloromethane. Then 723 mg (7.16 mmol) of triethylamine and 88 mg (0.72 mmol) of DMAP were added. Finally, a solution of methanesulfonic acid (3.95 mmol) in dichloromethane (5 ml) was added dropwise at around 0°C, and the mixture was stirred at room temperature overnight. When the reaction was monitored by TLC and found completed, the reaction was quenched by slow addition of water (20 ml). The organic phase was obtained by extraction with dichloromethane, dried, and concentrated to obtain a crude product. The product was dissolved in 8 ml of ethyl acetate, and cooled to induce crystallization, obtaining a pale-yellow solid (86.6% yield; 98.52% ee; purity 95.65%).

ESI-MS:m/z=319.1(M+H)⁺.

### Step 5: Preparation of Compound 1

200 mg (0.539 mmol) of 4-(5-chloro-2-(4-(trifluoromethyl)-1H-1,2,3-triazol-1-yl)phenyl)-5-methoxypyridin-2(1H)-one was added into a 25 ml single-neck flask, and dissolved in 5 ml of dioxane with stirring. 150 mg (1.08 mmol) of potassium carbonate and 62 mg (0.539 mmol) of tetramethylguanidine were added, and the mixture was stirred for 5 min. (R)-1-((4-amino-3-fluorophenyl)amino)-1-oxobutan-2-ylmethanesulfonate (0.648 mmol) was then introduced, after which the reaction mixture was warmed to 35 °C and maintained with stirring until completion of reaction was confirmed by TLC. The N/O-alkylation conversion ratio was 40:1. The reaction was quenched with the addition of saturated ammonium chloride, and extracted with ethyl acetate. The organic phase was washed sequentially with water, saturated brine, and anhydrous sodium sulfate, and the solvent was evaporated to obtain the crude product with 95.8% yield, and 99.78% ee.

ESI-MS:m/z=593.2(M+H)⁺.

¹H NMR (400 MHz, DMSO-d6) δ: 10.78 (s, 1H), 9.14 (s, 1H), 7.88 - 7.77 (m, 3H), 7.72 - 7.61 (m, 2H), 7.55 (d, 2H), 7.37 (dd, 1H), 7.13 (s, 1H), 6.54 (s, 1H), 5.52 (dd, 1H), 3.25 (s, 3H), 2.18 - 2.00 (m, 2H), 0.78 (t, 3H).

Crystallization of the crude product yielded a high-quality target compound.

### Example 24: Preparation of Compound 2

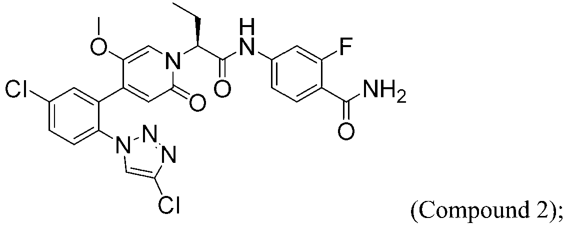

The preparation method was the same as that in Example 23, except that 4-(5-chloro-2-(4-(trifluoromethyl)-1H-1,2,3-triazol-1-yl)phenyl)-5-methoxypyridin-2(1H)-one in Step 5 was replaced with 4-(5-chloro-2-(4-chloro-1H-1,2,3-triazol-1-yl)phenyl)-5-methoxypyridin-2(1H)-one, i.e., R⁹ was converted from the trifluoromethyl to a chloro group, thereby obtaining the crude compound 2 in 90% yield and 98.61% ee. At the reaction endpoint, the N/O-alkylation conversion ratio was 35:1.

ESI-MS:m/z=559.1(M+H)⁺.

¹H NMR (400 MHz, DMSO-d6) δ:10.69 (s, 1H), 9.15 (d, 1H), 7.98 - 7.72 (m, 5H), 7.59 - 7.28 (m, 3H), 7.15 (s, 1H), 6.52 (s, 1H), 5.52 (dd 1H), 3.28 (s, 3H), 2.15-2.03 (m, 2H), 0.78 (t, 3H).

Crystallization of the crude product yielded a high-quality target compound.

### Example 25: Preparation of Compound 3

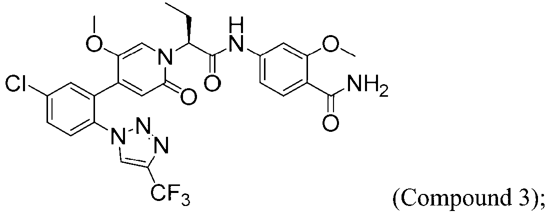

The preparation method was the same as in Example 23, except that 4-amino-2-fluorobenzamide in Step 2 was replaced with 4-amino-2-methoxy-benzamide, i.e., R⁶ was converted from the fluoro group to a methoxy group, thereby obtaining crude Compound 3 in 90% yield and 98.39% ee. At the reaction endpoint, the N/O-alkylation ratio was 30:1.

ESI-MS:m/z=605.1(M+H)⁺.

¹H NMR (400 MHz, DMSO-d6) δ:10.68 (s, 1H), 9.14 (d, 1H), 7.95 - 7.73 (m, 4H), 7.56 (m, 2H), 7.46 (d, 1H), 7.19 (dd, 1H), 7.14 (s, 1H), 6.53 (s, 1H), 5.54 (dd, 1H), 3.86 (s, 3H), 3.25 (s, 3H), 2.16-2.04 (m, 2H), 0.78 (t, 3H).

### Example 26: Preparation of Compound 4

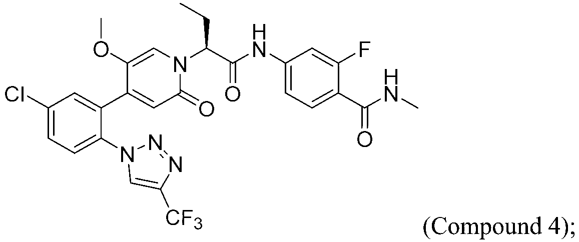

The preparation method was the same as in Example 23, except that 4-amino-2-fluorobenzamide in step 2 was replaced with 4-amino-2-fluoro-N-methylbenzamide, i.e., the R² was converted from the amino group to an N-methyl group, thereby obtaining the crude compound 4 in 90% yield and 99.02% ee. At the reaction endpoint, the N/O-alkylation ratio was 40:1.

ESI-MS:m/z=623.1(M+H)⁺.

¹H NMR (400 MHz, DMSO-d6) δ: 10.82 (s, 1H), 9.16 (d, J = 1.1 Hz, 1H), 8.13 - 8.06 (m, 1H), 7.89 - 7.80 (m, 2H), 7.79 (d, J = 2.0 Hz, 1H), 7.70 - 7.60 (m, 2H), 7.37 (dd, J = 8.5, 2.0 Hz, 1H), 7.13 (s, 1H), 6.54 (s, 1H), 5.52 (t, J = 7.8 Hz, 1H), 3.25 (s, 3H), 2.76 (d, J = 4.6 Hz, 3H), 2.18 - 2.02 (m, 2H), 0.78 (t, J = 7.2 Hz, 3H).

### Example 27: Preparation of Compound 5

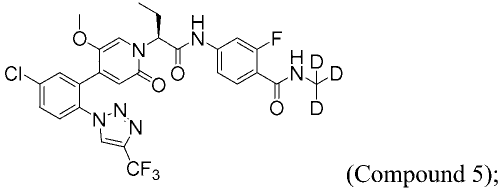

The preparation method was the same as in Example 23, except that 4-amino-2-fluorobenzamide in Step 2 was replaced with 4-amino-2-fluoro-N-(methyl-d3) benzamide, i.e., the R² was converted from the amino group to an N-(methyl-d3) group, thereby obtaining the crude compound 5 in 89% yield and 98.87% ee. At the reaction endpoint, the N/O-alkylation ratio was 40:1.

ESI-MS:m/z=610.2(M+H)⁺.

¹H NMR (400 MHz, DMSO-d6) δ: 10.79 (s, 1H), 9.14 (d, J = 1.1 Hz, 1H), 8.06 (d, J = 3.4 Hz, 1H), 7.92 - 7.81 (m, 2H), 7.81 - 7.76 (m, 1H), 7.70 - 7.60 (m, 2H), 7.37 (dd, J = 8.6, 2.0 Hz, 1H), 7.13 (s, 1H), 6.54 (s, 1H), 5.51 (d, J = 8.6 Hz, 1H), 3.25 (s, 3H), 2.19 - 1.99 (m, J = 7.1 Hz, 2H), 0.78 (t, J = 7.2 Hz, 3H).

### Example 28: Preparation of Compound 6

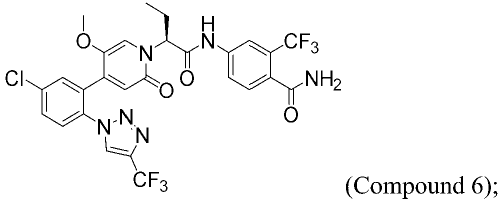

The preparation method was the same as in Example 23, except that 4-amino-2-fluorobenzamide in Step 2 was replaced with 4-amino-2-trifluoromethyl-N-methylbenzamide, i.e., R⁶ was converted from the fluoro group to a trifluoromethyl group, thereby obtaining crude compound 6 in 89% yield and 98.87% ee. At the reaction endpoint, the N/O-alkylation ratio was 30:1.

ESI-MS:m/z=643.1(M+H)⁺.

¹H NMR (400 MHz, DMSO-d6) δ:10.80 (s, 1H), 9.13 (s, 1H), 8.12 (d, 1H), 8.02 - 7.72 (m, 5H), 7.54-7.51 (m, 2H), 7.14 (s, 1H), 6.54 (s, 1H), 5.51 (dd, 1H), 3.25 (s, 3H), 2.23 - 2.03 (m, 2H), 0.79 (t, 3H).

### Example 29: Preparation of Compound 7

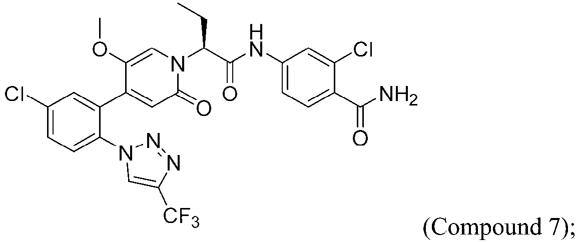

The preparation method was the same as in Example 23, except that 4-amino-2-fluorobenzamide in Step 2 was replaced with 4-amino-2-chloro-benzamide, i.e., R⁶ was converted from the fluoro group to a chloro group, thereby obtaining crude compound 7 in 92% yield and 98.87% ee. At the reaction endpoint, the N/O-alkylation ratio was 35:1.

ESI-MS:m/z=623.1(M+H)⁺.

¹H NMR (400 MHz, DMSO-d6) δ:10.58 (s, 1H), 9.11 (s, 1H), 7.91 - 7.66 (m, 4H), 7.58 - 7.25 (m, 3H), 7.15 (s, 1H), 6.51 (s, 1H), 5.53 (dd 1H), 3.26 (s, 3H), 2.88 (d, 3H),2.12-2.01 (m, 2H), 0.79 (t, 3H).

Table 4 lists the intermediates of the examples 23-29 described above together with their NMR and MS data.

**Table 4. Intermediates of the examples 23-29 described above together with their NMR and MS data.**

| Structure & ID | Name | ¹H NMR | ESI-MS:m/z (M+H)⁺ |
|---|---|---|---|
| 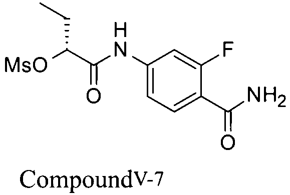 | (R)-1-((4-amin o-3-fluorophen yl)amino)-1-ox obutan-2-yl methanesulfona te | 1H NMR (400 MHz, DMSO-d6) δ: 10.76 (s, 1H), 8.08-7.97 (m, 1H), 7.92-7.88 (m, 1H), 7.76 (s, 2H), 7.60-7.56 (m, 1H), 4.56-4.52 (m, 1H), 3.26 (s, 3H), 1.98-1.88 (m, 2H), 0.88 (t, 3H). | 319.1 |
| 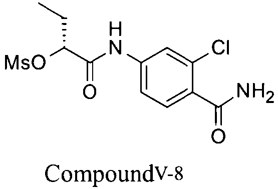 | (R)-1-((4-amin o-3-chlorophen yl)amino)-1-ox obutan-2-yl methanesulfona te | 1H NMR (400 MHz, DMSO-d6) δ: 10.76 (s, 1H), 8.16-8.08 (m, 1H), 7.98-7.92 (m, 1H), 7.79 (s, 2H), 7.60-7.55 (m, 1H), 4.56-4.52 (m, 1H), 3.22 (s, 3H), 1.96-1.88 (m, 2H), 0.87 (t, 3H). | 335.1 |
| 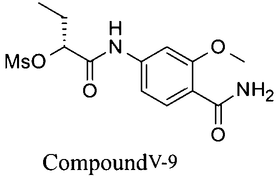 | (R)-1-((4-amin o-3-methoxyph enyl)amino)-1-oxobutan-2-yl methanesulfona te | 1H NMR (400 MHz, DMSO-d6) δ: 10.76 (s, 1H), 7.98-7.92 (m, 1H), 7.82-7.76 (m, 1H), 7.65 (s, 2H), 7.60-7.55 (m, 1H), 4.56-4.52 (m, 1H), 3.82 (s, 3H), 3.22 (s, 3H), 1.96-1.88 (m, 2H), 0.87 (t, 3H). | 331.1 |
| 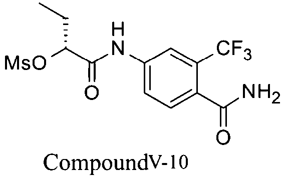 | (R)-1-((4-amin o-3-trifluorome thylphenyl)ami no)-1-oxobutan -2-yl methanesulfona te | 1H NMR (400 MHz, DMSO-d6) δ: 10.76 (s, 1H), 8.26-8.19 (m, 1H), 8.08-7.99 (m, 1H), 7.86 (s, 2H), 7.75-7.68 (m, 1H), 4.56-4.52 (m, 1H), 3.22 (s, 3H), 1.98-1.90(m, 2H), 0.86 (t, 3H). | 369.1 |
| 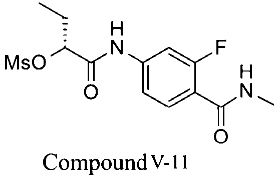 | (R)-1-((3-fluor o-4-(methylcar bamoyl)phenyl )amino)-1-oxob utan-2-yl methanesulfona te | 1H NMR (400 MHz, DMSO-d6) δ: 10.76 (s, 1H), 8.19 (s, 1H), 8.08-7.98 (m, 1H), 7.95-7.89 (m, 1H), 7.62-7.56 (m, 1H), 4.56-4.52 (m, 1H), 3.26 (s, 3H), 2.66 (s, 3H),1.98-1.88 (m, 2H), 0.88 (t, 3H). | 333.1 |
| 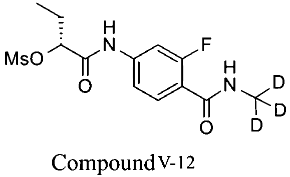 | (R)-1-((3-fluor o-4-((methyl-d 3)carbamoyl)p henyl)amino)-1-oxobutan-2-y 1 methanesulfona te | 1H NMR (400 MHz, DMSO-d6) δ: 10.76 (s, 1H), 8.18 (s, 1H), 8.08-7.98 (m, 1H), 7.96-7.89 (m, 1H), 7.63-7.55 (m, 1H), 4.56-4.52 (m, 1H), 3.26 (s, 3H), 1.98-1.88 (m, 2H), 0.88 (t, 3H). | 336.1 |

### Part 3. Third Set of Examples

Examples 30-36 provide a class of intermediates and the oxopyridine compounds of formula (I) synthesized therefrom, in which R⁷ is a p-nitrobenzenemethanesulfonyl group. The details are as follows:

### Example 30: Preparation of (S)-2-fluoro-4-(2-(4-(5-chloro-2-(4-(trifluoromethyl)-1H-1,2,3-triazol-1-yl)phenyl)-5-methoxy-2 -oxopyridin-1(2H)-yl)butanamido)benzamide (Compound 1):

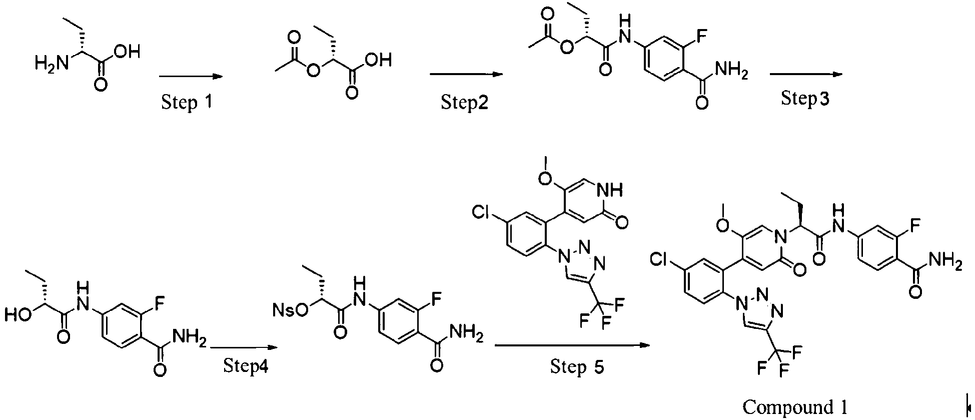

wherein, steps 1-3 and 5 are the same as in Example 16, except that:
Step 4: Preparation of (R)-1-((4-amino-3-fluorophenyl)amino)-1-oxobutan-2-yl p-nitrobenzenesulfonate.

860 mg (3.58 mmol) of (R)-2-fluoro-4-(2-hydroxybutanamido)benzamide was dissolved in 10 ml dichloromethane. Then 723 mg (7.16 mmol) of triethylamine and 88 mg (0.72 mmol) of DMAP were added. Finally, a solution of 802 mg (3.95 mmol) p-nitrobenzenesulfonic acid in dichloromethane (5 ml) was added dropwise at approximately 0°C, and the mixture was stirred at room temperature overnight. When the reaction was monitored by TLC and found completed, the reaction was quenched by slow addition of water (20 ml). The organic phase extracted with dichloromethane was dried, and concentrated to obtain a crude product. The product was dissolved in 8 ml of ethyl acetate, and cooled to induce crystallization, obtaining a pale-yellow solid (86.6% yield; 98.52% ee; purity 95.65%).

ESI-MS:m/z=426.1(M+H)⁺.

### Step 5: Preparation of Compound 1

200 mg (0.539 mmol) of 4-(5-chloro-2-(4-(trifluoromethyl)-1H-1,2,3-triazol-1-yl)phenyl)-5-methoxypyridin-2(1H)-one was added into a 25 ml single-neck flask, and dissolved in 5 ml of dioxane with stirring. 150 mg (1.09 mmol) of potassium carbonate and 62 mg (0.539 mmol) of tetramethylguanidine were added, and the mixture was stirred for 5 min. (R)-1-((4-amino-3-fluorophenyl)amino)-1-oxobutan-2-yl p-nitrobenzenesulfonate (0.648 mmol) was then introduced, after which the reaction mixture was warmed to 35 °C and maintained with stirring until completion of the reaction was confirmed by TLC. The N/O-alkylation conversion ratio was 40:1. The reaction was quenched with the addition of saturated ammonium chloride, and extracted with ethyl acetate. The organic phase was washed sequentially with water, saturated brine, and anhydrous sodium sulfate, and the solvent was evaporated to obtain the crude product with 95.8% yield, and 98.51% ee.

ESI-MS:m/z=593.2(M+H)⁺.

¹H NMR (400 MHz, DMSO-d6) δ: 10.78 (s, 1H), 9.14 (s, 1H), 7.88 - 7.77 (m, 3H), 7.72 - 7.61 (m, 2H), 7.55 (d, 2H), 7.37 (dd, 1H), 7.13 (s, 1H), 6.54 (s, 1H), 5.52 (dd, 1H), 3.25 (s, 3H), 2.18 - 2.00 (m, 2H), 0.78 (t, 3H).

Crystallization of the crude product yielded a high-quality target compound.

### Example 31: Preparation of Compound 2

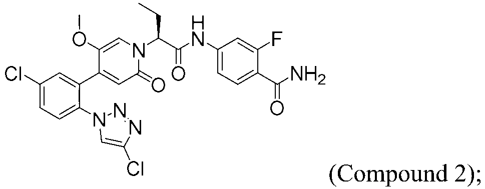

The preparation method was the same as that in Example 30, except that 4-(5-chloro-2-(4-(trifluoromethyl)-1H-1,2,3-triazol-1-yl)phenyl)-5-methoxypyridin-2(1H)-one in Step 5 was replaced with 4-(5-chloro-2-(4-chloro-1H-1,2,3-triazol-1-yl)phenyl)-5-methoxypyridin-2(1H)-one, i.e., R⁹ was converted from the trifluoromethyl to a chloro group, thereby obtaining the crude compound 2 in 90% yield and 98.61% ee. At the reaction endpoint, the N/O-alkylation conversion ratio was 35:1.

ESI-MS:m/z=559.1(M+H)⁺.

¹H NMR (400 MHz, DMSO-d6) δ:10.69 (s, 1H), 9.15 (d, 1H), 7.98 - 7.72 (m, 5H), 7.59 - 7.28 (m, 3H), 7.15 (s, 1H), 6.52 (s, 1H), 5.52 (dd 1H), 3.28 (s, 3H), 2.15-2.03 (m, 2H), 0.78 (t, 3H).

Crystallization of the crude product yielded a high-quality target compound.

### Example 32: Preparation of Compound 3

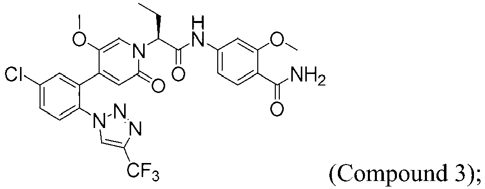

The preparation method was the same as in Example 30, except that 4-amino-2-fluorobenzamide in Step 2 was replaced with 4-amino-2-methoxy-benzamide, i.e., R⁶ was converted from the fluoro group to a methoxy group, thereby obtaining crude Compound 3 in 90% yield and 98.39% ee. At the reaction endpoint, the N/O-alkylation ratio was 30:1.

ESI-MS:m/z=605.1(M+H)⁺.

¹H NMR (400 MHz, DMSO-d6) δ:10.68 (s, 1H), 9.14 (d, 1H), 7.95 - 7.73 (m, 4H), 7.56 (m, 2H), 7.46 (d, 1H), 7.19 (dd, 1H), 7.14 (s, 1H), 6.53 (s, 1H), 5.54 (dd, 1H), 3.86 (s, 3H), 3.25 (s, 3H), 2.16-2.04 (m, 2H), 0.78 (t, 3H).

### Example 33: Preparation of Compound 4

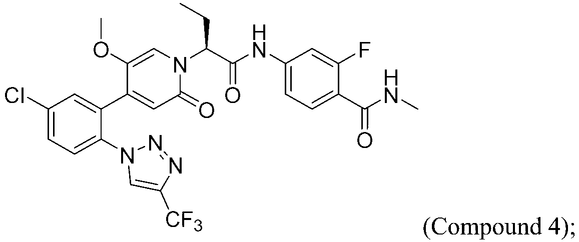

The **preparation** method was the same as in Example 30, except that 4-amino-2-fluorobenzamide in step 2 was replaced with 4-amino-2-fluoro-N-methylbenzamide, i.e., the R² was converted from the amino group to an N-methyl group, thereby obtaining the crude compound 4 in 90% yield and 99.02% ee. At the reaction endpoint, the N/O-alkylation ratio was 40:1.

ESI-MS:m/z=623.1(M+H)⁺.

¹H NMR (400 MHz, DMSO-d6) δ: 10.82 (s, 1H), 9.16 (d, J = 1.1 Hz, 1H), 8.13 - 8.06 (m, 1H), 7.89 - 7.80 (m, 2H), 7.79 (d, J = 2.0 Hz, 1H), 7.70 - 7.60 (m, 2H), 7.37 (dd, J = 8.5, 2.0 Hz, 1H), 7.13 (s, 1H), 6.54 (s, 1H), 5.52 (t, J = 7.8 Hz, 1H), 3.25 (s, 3H), 2.76 (d, J = 4.6 Hz, 3H), 2.18 - 2.02 (m, 2H), 0.78 (t, J = 7.2 Hz, 3H).

### Example 34: Preparation of Compound 5

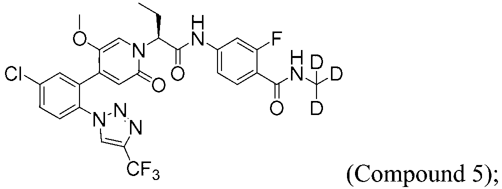

The preparation method was the same as in Example 30, except that 4-amino-2-fluorobenzamide in Step 2 was replaced with 4-amino-2-fluoro-N-(methyl-d3) benzamide, i.e., the R² was converted from the amino group to an N-(methyl-d3) group, thereby obtaining the crude compound 5 in 89% yield and 98.87% ee. At the reaction endpoint, the N/O-alkylation ratio was 40:1.

ESI-MS:m/z=610.2(M+H)⁺.

¹H NMR (400 MHz, DMSO-d6) δ: 10.79 (s, 1H), 9.14 (d, J = 1.1 Hz, 1H), 8.06 (d, J = 3.4 Hz, 1H), 7.92 - 7.81 (m, 2H), 7.81 - 7.76 (m, 1H), 7.70 - 7.60 (m, 2H), 7.37 (dd, J = 8.6, 2.0 Hz, 1H), 7.13 (s, 1H), 6.54 (s, 1H), 5.51 (d, J = 8.6 Hz, 1H), 3.25 (s, 3H), 2.19 - 1.99 (m, J = 7.1 Hz, 2H), 0.78 (t, J = 7.2 Hz, 3H).

### Example 35: Preparation of Compound 6

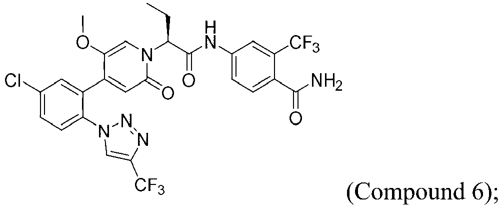

The preparation method was the same as in Example 30, except that 4-amino-2-fluorobenzamide in Step 2 was replaced with 4-amino-2-trifluoromethyl-N-methylbenzamide, i.e., R⁶ was converted from the fluoro group to a trifluoromethyl group, thereby obtaining crude compound 6 in 89% yield and 98.87% ee. At the reaction endpoint, the N/O-alkylation ratio was 30:1.

ESI-MS:m/z=643.1(M+H)⁺.

¹H NMR (400 MHz, DMSO-d6) δ:10.80 (s, 1H), 9.13 (s, 1H), 8.12 (d, 1H), 8.02 - 7.72 (m, 5H), 7.54-7.51 (m, 2H), 7.14 (s, 1H), 6.54 (s, 1H), 5.51 (dd, 1H), 3.25 (s, 3H), 2.23 - 2.03 (m, 2H), 0.79 (t, 3H).

### Example 36: Preparation of Compound 7

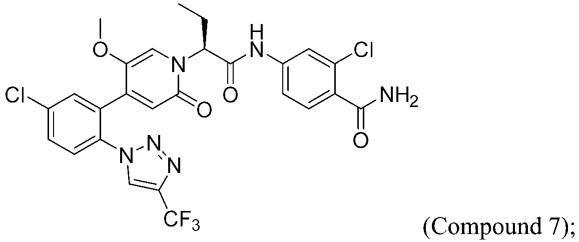

The preparation method was the same as in Example 30, except that 4-amino-2-fluorobenzamide in Step 2 was replaced with 4-amino-2-chlorobenzamide, i.e., R⁶ was converted from the fluoro group to a chloro group, thereby obtaining crude compound 7 in 92% yield and 98.87% ee. At the reaction endpoint, the N/O-alkylation ratio was 35:1.

ESI-MS:m/z=623.1(M+H)⁺.

¹H NMR (400 MHz, DMSO-d6) δ:10.58 (s, 1H), 9.11 (s, 1H), 7.91 - 7.66 (m, 4H), 7.58 - 7.25 (m, 3H), 7.15 (s, 1H), 6.51 (s, 1H), 5.53 (dd 1H), 3.26 (s, 3H), 2.88 (d, 3H),2.12-2.01 (m, 2H), 0.79 (t, 3H).

Table 5 lists the intermediates of the examples 30-36 described above together with their NMR and MS data.

**Table 5. Intermediates of the examples 30-36 described above together with their NMR and MS data.**

| Structure & ID | Name | ¹H NMR | ESI-M S:m/z (M+H)⁺ |
|---|---|---|---|
| 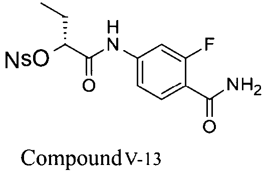 | (R)-1-((4-ami no-3-fluorophenyl )amino)-1-oxobuta n-2-yl p-nitrobenzenesulf onate | ¹H NMR (400 MHz, DMSO-d6) δ: 10.28 (s, 1H), 8.52-8.46 (m, 2H), 8.12-7.90 (m, 4H), 7.78 (s, 2H), 7.60-7.56 (m, 1H), 5.56-5.54 (m, 1H), 2.01-1.88 (m, 2H), 0.88 (t, 3H). | 426.1 |
| 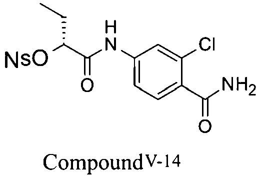 | (R)-1-((4-ami no-3-chlorophenyl )amino)-1-oxobuta n-2-yl p-nitrobenzenesulf onate | ¹H NMR (400 MHz, DMSO-d6) δ: 10.26 (s, 1H), 8.52 -8.45(m, 2H), 8.22-8.16 (m,4H), 7.82 (s, 2H), 7.60-7.56 (m, 1H), 5.56-5.53 (m, 1H), 2.01-1.88 (m, 2H), 0.88 (t, 3H). | 442.1 |
| 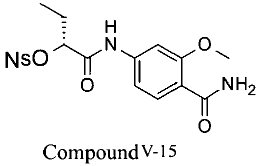 | (R)-1-((4-ami no-3-methoxyphenyl)a mino)-1-oxobutan-2-yl p-nitrobenzenesulf onate | ¹H NMR (400 MHz, DMSO-d6) δ: 10.28 (s, 1H), 8.52-8.46 (m, 2H), 7.99-7.90 (m, 4H), 7.65 (s, 2H), 7.55-7.46 (m, 1H), 5.55-5.52 (m, 1H), 3.96 (s,3H), 2.01-1.88 (m, 2H), 0.88 (t, 3H). | 438.1 |
| 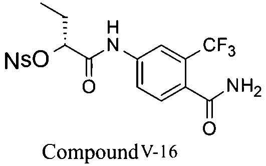 | (R)-1-((4-ami no-3-trifluorometh ylphenyl)amino)-1 -oxobutan-2-yl p-nitrobenzenesulf onate | ¹H NMR (400 MHz, DMSO-d6) δ: 10.26 (s, 1H), 8.51-8.45 (m, 2H), 8.32-8.26 (m,4H), 7.82 (s, 2H), 7.60-7.56 (m, 1H), 5.56-5.53 (m, 1H), 2.00-1.88(m,2H),0.86 (t, 3H). | 476.1 |
| 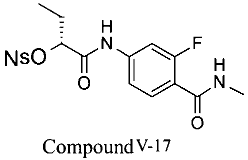 | (R)-1-((3-fluo ro-4-(methylcarba moyl)phenyl)amin o)-1-oxobutan-2-y l p-nitrobenzenesulf onate | ¹H NMR (400 MHz, DMSO-d6) δ: 10.28 (s, 1H), 8.51-8.46 (m, 2H), 8.38 (s, 1H), 8.12-7.90 (m, 4H), 7.60-7.56 (m, 1H), 5.56-5.52 (m, 1H), 2.81 (s, 3H), 2.01-1.88 (m, 2H), 0.88 (t, 3H). | 440.1 |
| 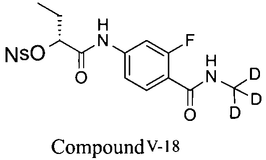 | (R)-1-((3-fluo ro-4-((methyl-d3)c arbamoyl)phenyl)a mino)-1-oxobutan-2-yl p-nitrobenzenesulf onate | ¹H NMR (400 MHz, DMSO-d6) δ: 10.28 (s, 1H), 8.52-8.45 (m, 2H), 8.38 (s, 1H), 8.12-7.90 (m, 4H), 7.60-7.56 (m, 1H), 5.56-5.52 (m, 1H), 2.01-1.88 (m, 2H), 0.88 (t, 3H). | 443.1 |

### Part 3. Forth Set of Examples

Examples 37-43 provide a class of intermediates and the oxopyridine compounds of formula (I) synthesized therefrom, in which R⁷ is a benzenesulfonyl group. The details are as follows:

### Example 37: Preparation of (S)-2-fluoro-4-(2-(4-(5-chloro-2-(4-(trifluoromethyl)-1H-1,2,3-triazol-1-yl)phenyl)-5-methoxy-2 -oxopyridin-1(2H)-yl)butanamido)benzamide (Compound 1):

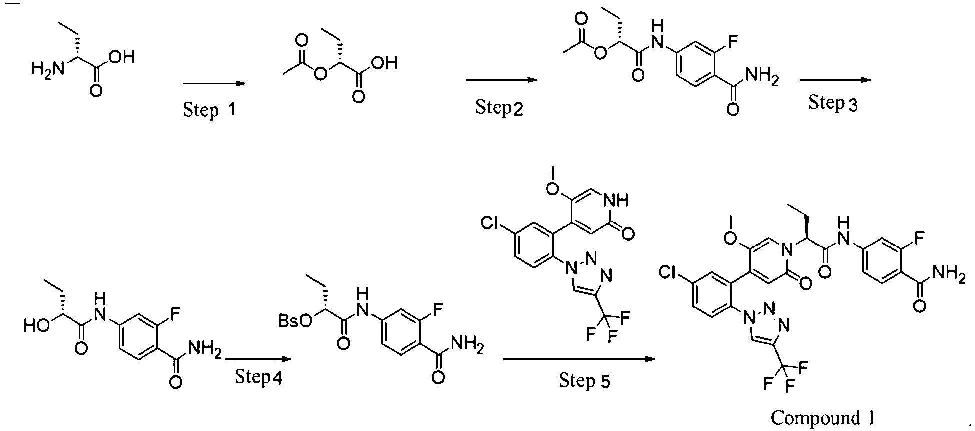

wherein, steps 1-3 and 5 are the same as in Example 16, except that:
Step 4: Preparation of (R)-1-((4-amino-3-fluorophenyl)amino)-1-oxobutan-2-yl methanesulfonate
860 mg (3.58 mmol) of (R)-2-fluoro-4-(2-hydroxybutanamido)benzamide was dissolved in 10 ml dichloromethane. Then 723 mg (7.16 mmol) of triethylamine and 88 mg (0.72 mmol) of DMAP were added. Finally, a solution of 625 mg (3.95 mmol) benzenesulfonic acid in dichloromethane (5 ml) was added dropwise at approximately 0°C, and the mixture was stirred at room temperature overnight. When the reaction was monitored by TLC and found completed, the reaction was quenched by slow addition of water (20 ml). The organic phase was obtained by extraction with dichloromethane, dried, and concentrated to obtain a crude product. The product was dissolved in 8 ml of ethyl acetate, and cooled to induce crystallization, obtaining a pale-yellow solid (86.6% yield; 98.52% ee; purity 95.65%).

ESI-MS:m/z=381.1(M+H)⁺.

### Step 5: Preparation of Compound 1

200 mg (0.539 mmol) of 4-(5-chloro-2-(4-(trifluoromethyl)-1H-1,2,3-triazol-1-yl)phenyl)-5-methoxypyridin-2(1H)-one was added into a 25 ml single-neck flask, and dissolved in 5 ml of dioxane with stirring. 150 mg (1.08 mmol) of potassium carbonate and 62 mg (0.539 mmol) of tetramethylguanidine were added, and the mixture was stirred for 5 min. 246 mg (0.648 mmol) of (R)-1-((4-amino-3-fluorophenyl)amino)-1-oxobutan-2-yl benzenesulfonate was then introduced, after which the reaction mixture was warmed to 35 °C and maintained with stirring until completion of the reaction was confirmed by TLC. The N/O-alkylation conversion ratio was 40:1. The reaction was quenched with the addition of saturated ammonium chloride, and extracted with ethyl acetate. The organic phase was washed sequentially with water, saturated brine, and anhydrous sodium sulfate, and the solvent was evaporated to obtain the crude product with 92.8% yield, and 98.51% ee.

ESI-MS:m/z=593.2(M+H)⁺.

¹H NMR (400 MHz, DMSO-d6) δ: 10.78 (s, 1H), 9.14 (s, 1H), 7.88 - 7.77 (m, 3H), 7.72 - 7.61 (m, 2H), 7.55 (d, 2H), 7.37 (dd, 1H), 7.13 (s, 1H), 6.54 (s, 1H), 5.52 (dd, 1H), 3.25 (s, 3H), 2.18 - 2.00 (m, 2H), 0.78 (t, 3H).

Crystallization of the crude product yielded a high-quality target compound.

### Example 38: Preparation of Compound 2

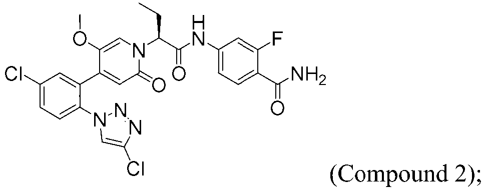

The preparation method was the same as that in Example 37, except that 4-(5-chloro-2-(4-(trifluoromethyl)-1H-1,2,3-triazol-1-yl)phenyl)-5-methoxypyridin-2(1H)-one in Step 5 was replaced with 4-(5-chloro-2-(4-chloro-1H-1,2,3-triazol-1-yl)phenyl)-5-methoxypyridin-2(1H)-one, i.e., R⁹ was converted from the trifluoromethyl to a chloro group, thereby obtaining the crude compound 2 in 90% yield and 98.61% ee. At the reaction endpoint, the N/O-alkylation conversion ratio was 35:1.

ESI-MS:m/z=559.1(M+H)⁺.

¹H NMR (400 MHz, DMSO-d6) δ:10.69 (s, 1H), 9.15 (d, 1H), 7.98 - 7.72 (m, 5H), 7.59 - 7.28 (m, 3H), 7.15 (s, 1H), 6.52 (s, 1H), 5.52 (dd 1H), 3.28 (s, 3H), 2.15-2.03 (m, 2H), 0.78 (t, 3H).

Crystallization of the crude product yielded a high-quality target compound.

### Example 39: Preparation of Compound 3

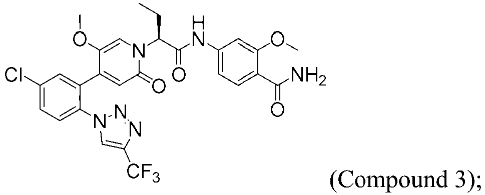

The preparation method was the same as in Example 37, except that 4-amino-2-fluorobenzamide in Step 2 was replaced with 4-amino-2-methoxy-benzamide, i.e., R⁶ was converted from the fluoro group to a methoxy group, thereby obtaining crude Compound 3 in 90% yield and 98.39% ee. At the reaction endpoint, the N/O-alkylation ratio was 30:1.

ESI-MS:m/z=605.1(M+H)⁺.

¹H NMR (400 MHz, DMSO-d6) δ:10.68 (s, 1H), 9.14 (d, 1H), 7.95 - 7.73 (m, 4H), 7.56 (m, 2H), 7.46 (d, 1H), 7.19 (dd, 1H), 7.14 (s, 1H), 6.53 (s, 1H), 5.54 (dd, 1H), 3.86 (s, 3H), 3.25 (s, 3H), 2.16-2.04 (m, 2H), 0.78 (t, 3H).

### Example 40: Preparation of Compound 4

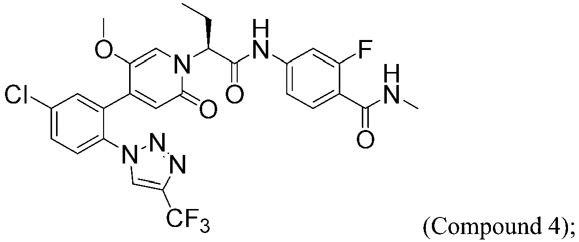

The preparation method was the same as in Example 37, except that 4-amino-2-fluorobenzamide in step 2 was replaced with 4-amino-2-fluoro-N-methylbenzamide, i.e., the R² was converted from the amino group to an N-methyl group, thereby obtaining the crude compound 4 in 90% yield and 99.02% ee. At the reaction endpoint, the N/O-alkylation ratio was 40:1.

ESI-MS:m/z=623.1(M+H)⁺.

¹H NMR (400 MHz, DMSO-d6) δ: 10.82 (s, 1H), 9.16 (d, J = 1.1 Hz, 1H), 8.13 - 8.06 (m, 1H), 7.89 - 7.80 (m, 2H), 7.79 (d, J = 2.0 Hz, 1H), 7.70 - 7.60 (m, 2H), 7.37 (dd, J = 8.5, 2.0 Hz, 1H), 7.13 (s, 1H), 6.54 (s, 1H), 5.52 (t, J = 7.8 Hz, 1H), 3.25 (s, 3H), 2.76 (d, J = 4.6 Hz, 3H), 2.18 - 2.02 (m, 2H), 0.78 (t, J = 7.2 Hz, 3H).

### Example 41: Preparation of Compound 5

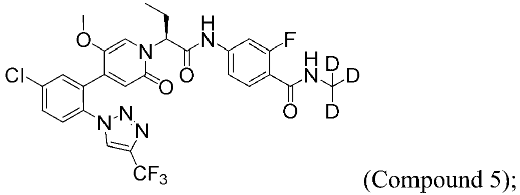

The preparation method was the same as in Example 37, except that 4-amino-2-fluorobenzamide in Step 2 was replaced with 4-amino-2-fluoro-N-(methyl-d3) benzamide, i.e., the R² was converted from the amino group to an N-(methyl-d3) group, thereby obtaining the crude compound 5 in 89% yield and 98.87% ee. At the reaction endpoint, the N/O-alkylation ratio was 40:1.

ESI-MS:m/z=610.2(M+H)⁺.

¹H NMR (400 MHz, DMSO-d6) δ: 10.79 (s, 1H), 9.14 (d, J = 1.1 Hz, 1H), 8.06 (d, J = 3.4 Hz, 1H), 7.92 - 7.81 (m, 2H), 7.81 - 7.76 (m, 1H), 7.70 - 7.60 (m, 2H), 7.37 (dd, J = 8.6, 2.0 Hz, 1H), 7.13 (s, 1H), 6.54 (s, 1H), 5.51 (d, J = 8.6 Hz, 1H), 3.25 (s, 3H), 2.19 - 1.99 (m, J = 7.1 Hz, 2H), 0.78 (t, J = 7.2 Hz, 3H).

### Example 42: Preparation of Compound 6

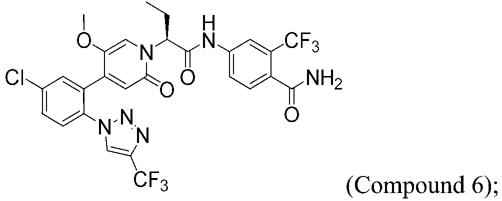

The preparation method was the same as in Example 37, except that 4-amino-2-fluorobenzamide in Step 2 was replaced with 4-amino-2-trifluoromethyl-N-methylbenzamide, i.e., R⁶ was converted from the fluoro group to a trifluoromethyl group, thereby obtaining crude compound 6 in 89% yield and 98.87% ee. At the reaction endpoint, the N/O-alkylation ratio was 30:1.

ESI-MS:m/z=643.1(M+H)⁺.

¹H NMR (400 MHz, DMSO-d6) δ:10.80 (s, 1H), 9.13 (s, 1H), 8.12 (d, 1H), 8.02 - 7.72 (m, 5H), 7.54-7.51 (m, 2H), 7.14 (s, 1H), 6.54 (s, 1H), 5.51 (dd, 1H), 3.25 (s, 3H), 2.23 - 2.03 (m, 2H), 0.79 (t, 3H).

### Example 43: Preparation of Compound 7

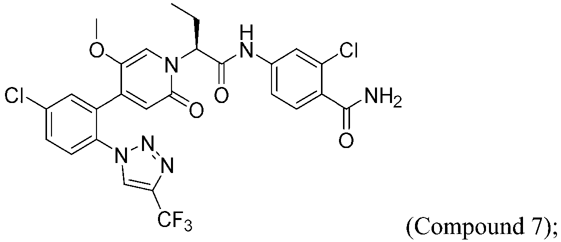

The preparation method was the same as in Example 37. 4-amino-2-fluorobenzamide in Step 2 was replaced with 4-amino-2-chlorobenzamide, i.e., R6 was converted from the fluoro group to a chloro group, thereby obtaining crude compound 7 in 92% yield and 98.87% ee. At the reaction endpoint, the N/O-alkylation ratio was 35:1.

ESI-MS:m/z=623.1(M+H)⁺.

¹H NMR (400 MHz, DMSO-d6) δ:10.58 (s, 1H), 9.11 (s, 1H), 7.91 - 7.66 (m, 4H), 7.58 - 7.25 (m, 3H), 7.15 (s, 1H), 6.51 (s, 1H), 5.53 (dd 1H), 3.26 (s, 3H), 2.88 (d, 3H),2.12-2.01 (m, 2H), 0.79 (t, 3H).

Table 6 lists the intermediates of the examples 37-43 described above together with their NMR and MS data;

**Table 6. Intermediates of the examples 37-43 described above together with their NMR and MS data.**

| Structure & ID | Name | ¹H NMR | ESI-MS:m/ z (M+H)⁺ |
|---|---|---|---|
| 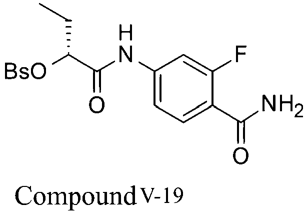 | (R)-1-((4-amin o-3-fluorophen yl)amino)-1-ox obutan-2-yl benzenesulfona te | ¹H NMR (400 MHz, DMSO-d6) δ: 10.28 (s, 1H), 8.07-7.96 (m, 4H), 7.92-7.86 (m, 3H), 7.82(s, 2H), 7.79-7.73 (m, 1H), 5.56-5.54 (m, 1H), 2.01-1.88 (m, 2H), 0.88 (t, 3H). | 381.1 |
| 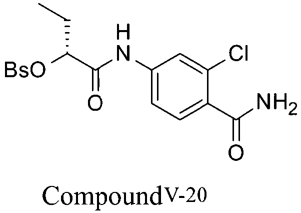 | (R)-1-((4-amin o-3-chlorophen yl)amino)-1-ox obutan-2-yl benzenesulfona te | ¹H NMR (400 MHz, DMSO-d6) δ: 10.26 (s, 1H), 8.17-8.06 (m, 3H), 8.02-7.96 (m, 2H), 7.92-7.86 (m, 2H), 7.82(s, 2H), 7.79-7.73 (m, 1H), 5.56-5.54 (m, 1H), 2.01-1.88 (m, 2H), 0.88 (t, 3H). | 397.1 |
| 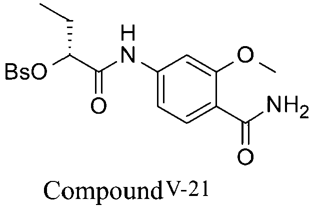 | (R)-1-((4-amin o-3-methoxyph enyl)amino)-1-oxobutan-2-yl benzenesulfona te | ¹H NMR (400 MHz, DMSO-d6) δ: 10.26 (s, 1H), 8.02-7.96 (m, 3H), 7.92-7.87 (m, 2H), 7.82(s, 2H), 7.62-7.56 (m, 2H), 7.79-7.73 (m, 1H), 5.56-5.54 (m, 1H), 3.92(s, 3H), 2.01-1.88 (m, 2H), 0.88 (t, 3H). | 393.1 |
| 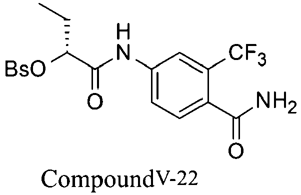 | (R)-1-((4-amin o-3-trifluorome thylphenyl)ami no)-1-oxobutan -2-yl benzenesulfona te | ¹H NMR (400 MHz, DMSO-d6) δ: 10.26 (s, 1H), 8.27-8.20 (m, 2H), 8.12-8.06 (m, 1H), 8.02-7.96 (m, 2H), 7.92-7.86 (m, 2H), 7.83(s, 2H), 7.79-7.73 (m, 1H), 5.56-5.54 (m, 1H), 2.01-1.88 (m, 2H), 0.88 (t, 3H). | 432.1 |
| 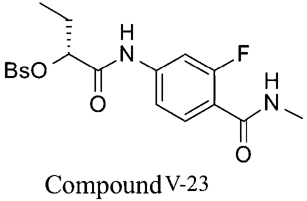 | (R)-1-((3-fluor o-4-(methylcar bamoyl)phenyl )amino)-1-oxob utan-2-yl benzenesulfona te | ¹H NMR (400 MHz, DMSO-d6) δ: 10.28 (s, 1H), 8.26(s, 1H), 8.07-7.96 (m, 4H), 7.92-7.86 (m, 3H), 7.79-7.73 (m, 1H), 5.56-5.54 (m, 1H), 2.82 (s, 3H), 2.01-1.88 (m, 2H), 0.88 (t, 3H). | 395.1 |
| 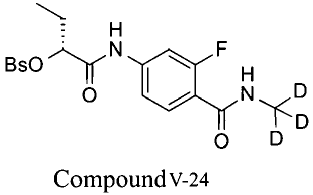 | (R)-1-((3-fluor o-4-((methyl-d 3)carbamoyl)p henyl)amino)-1 -oxobutan-2-yl benzenesulfona te | ¹H NMR (400 MHz, DMSO-d6) δ: 10.28 (s, 1H), 8.26(s, 1H), 8.07-7.96 (m, 4H), 7.92-7.86 (m, 3H), 7.79-7.73 (m, 1H), 5.56-5.54 (m, 1H), 2.01-1.88 (m, 2H), 0.88 (t, 3H). | 398.1 |

The embodiments described above are merely one of the preferred embodiments, and are not intended to limit the scope of the invention. Modifications or refinements made in keeping with the inventive concept and spirit, which do not materially affect the substance and address the same technical problem, are deemed to fall within the scope of the present invention.

## Claims

1. An intermediate of Formula (II) or a pharmaceutically acceptable salt thereof: 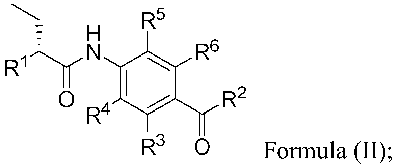 in Formula (II):
R¹ is selected from TsO-, X, and R⁷O-; wherein Ts is p-toluenesulfonyl, and X is selected from F, Cl, Br, and I;
R² is selected from -NHR⁸, and -OC(CH₃)₃;
R³, R⁴, R⁵, and R⁶ are each independently selected from hydrogen, halogen, alkoxy, and haloalkyl;
R⁷ is selected from trifluoromethanesulfonyl, methanesulfonyl, p-nitrobenzenemethanesulfonyl, and benzenesulfonyl;
R⁸ is selected from hydrogen, alkyl, and cycloalkyl;
provided that Formula (II) is not

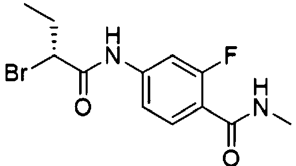

2. The intermediate according to claim 1, or a pharmaceutically acceptable salt thereof, wherein the intermediate has a structure represented by formula (III), (IV) or (V): 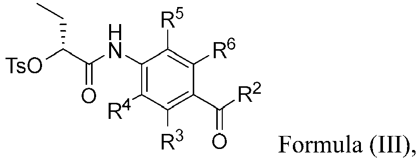 in Formula (III),
R² is selected from -NHR⁸, and R⁸ is selected from hydrogen, alkyl and cycloalkyl;
R³, R⁴, R⁵, and R⁶ are independently selected from hydrogen, halogen, alkoxy, and haloalkyl; 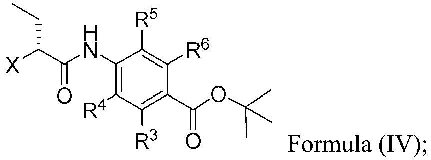 in Formula (IV):
R³, R⁴, R⁵, and R⁶ are each independently selected from hydrogen, halogen, alkoxy, and haloalkyl;
X is selected form F, Cl, Br, and I; 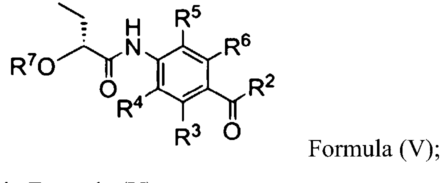 in Formula (V):
R² is selected from NHR⁸, and R⁸ is selected from hydrogen, alkyl and cycloalkyl;
R³, R⁴, R⁵, and R⁶ are independently selected from hydrogen, halogen, alkoxy, and haloalkyl;
R⁷ is selected from trifluoromethanesulfonyl, methanesulfonyl, p-nitrobenzenemethanesulfonyl and benzenesulfonyl.

3. The intermediate according to claim 2, or a pharmaceutically acceptable salt thereof, wherein,
in Formula (III),
R² is selected from -NHR⁸, and R⁸ is selected from hydrogen, methyl, ethyl, propyl, cyclopropyl, cyclopropylmethyl and tert-butyl;
and/or R³, R⁴, R⁵, and R⁶ are independently selected from hydrogen, fluoro, chloro, methoxy, ethoxy and trifluoromethyl;
In Formula (IV),
R³, R⁴, R⁵, and R⁶ are independently selected from hydrogen, fluoro, chloro, methoxy, ethoxy and trifluoromethyl,
and/or X is independently selected from Cl and Br;
in Formula (V),
R⁸ is selected from hydrogen, methyl, ethyl, propyl, cyclopropyl, cyclopropylmethyl and tert-butyl;
and/or R³, R⁴, R⁵, and R⁶ are independently selected from hydrogen, fluoro, chloro, methoxy, ethoxy and trifluoromethyl.

4. The intermediate according to claim 3, or a pharmaceutically acceptable salt thereof, wherein the intermediate has a structure represented by Formula (IV-a): 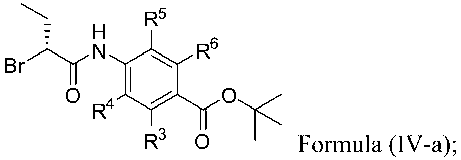 in Formula (IV-a), R³, R⁴, R⁵, and R⁶ are defined the same as in claim 3.

5. The intermediate according to claim 2, or a pharmaceutically acceptable salt thereof, wherein, at least one hydrogen atom in the intermediate is replaced by deuterium.

6. The intermediate according to claim 2, or a pharmaceutically acceptable salt thereof, wherein the intermediate comprises the following compounds:

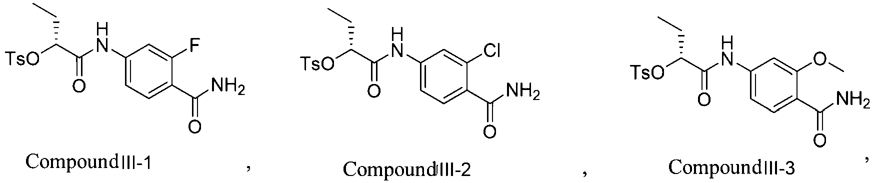

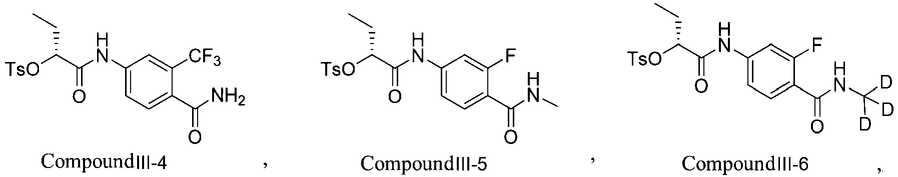

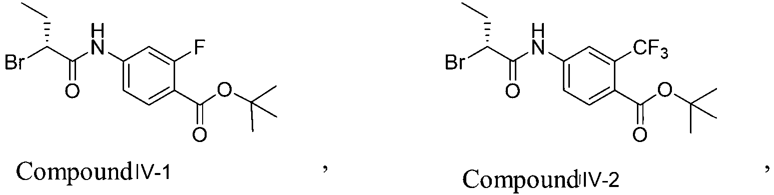

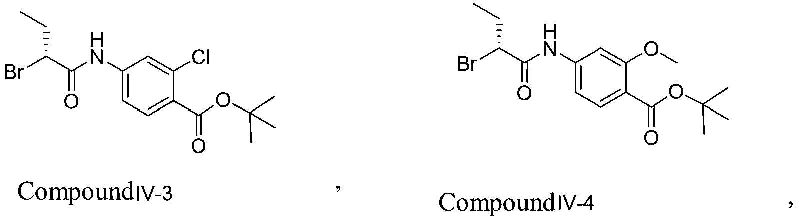

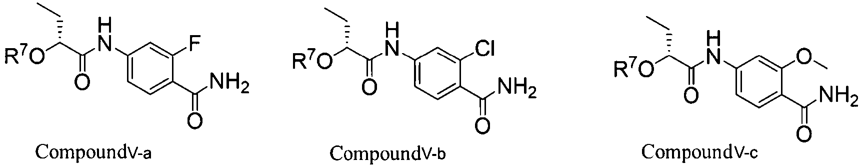

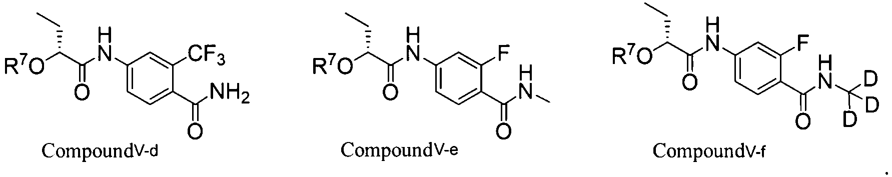

7. A method for preparing the intermediate according to any one of claims 1-6, or a pharmaceutically acceptable salt thereof, wherein the synthesis of the intermediate comprises Method A, Method B, or Method C:
Method A: the compound of Formula (III-a) is reacted with p-toluenesulfonyl chloride under a basic condition to give the intermediate of Formula (III), 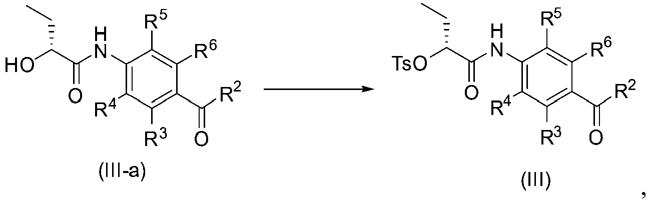 wherein, R², R³, R⁴, R⁵, and R⁶ are defined the same as in any of claims 1-6;
Method B: the compound of Formula (III-a-5) is reacted with halogenated carboxylic acid to give the intermediate of Formula (IV), 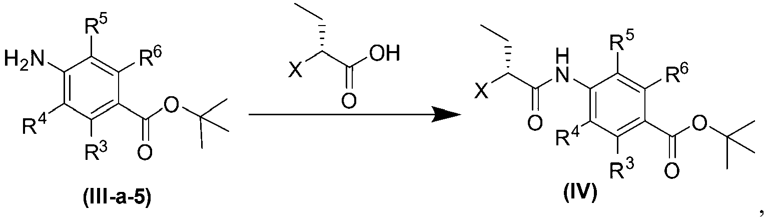 wherein, X is selected form F, Cl, Br and I; R³, R⁴, R⁵, and R⁶ are defined the same as in any of claims 1-6;
Method C: the compound of Formula (III-a) undergoes esterification with a hydroxyl-protecting reagent under a basic condition to give the intermediate of Formula (V), 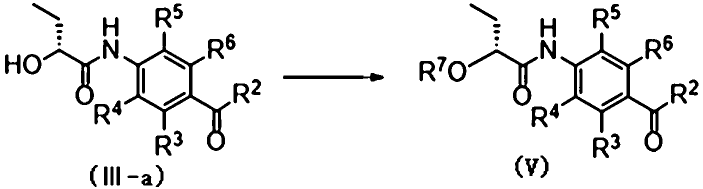 wherein, R², R³, R⁴, R⁵, R⁶, and R⁷ are defined the same as in any of claims 1-6.

8. The method according to claim 7, wherein Method A and/or Method C comprise the following reaction conditions:
the base is selected from organic bases;
the reaction solvent in the synthetic step is selected from organic solvents;
optionally, the molar ratio of the compound of Formula (III-a) to the organic base in the synthesis step is 1:0.5-8;
optionally, the reaction temperature of the synthesis step is 0-60 °C;
optionally, the reaction time of the synthesis step is 1-10 h.

9. The method according to claim 8, wherein
the organic base comprises any one of triethylamine, pyridine, tetramethylguanidine, DMAP, DBU, and DIPEA, or a mixture of two or more thereof;
the organic solvent comprises, but is not limited to, any one of dichloromethane, tetrahydrofuran, 2-methyltetrahydrofuran, isopropanol, ethanol, acetone, DMF, and acetonitrile, or a mixture of two or more thereof;
or the molar ratio of the compound of Formula (III-a) to the organic base is 1:1-3;
or the reaction temperature is 10-30 °C;
or the reaction time is 4-6 h.

10. The method according to claim 7, wherein the preparation of the compound of formula (III-a) comprises the following steps: 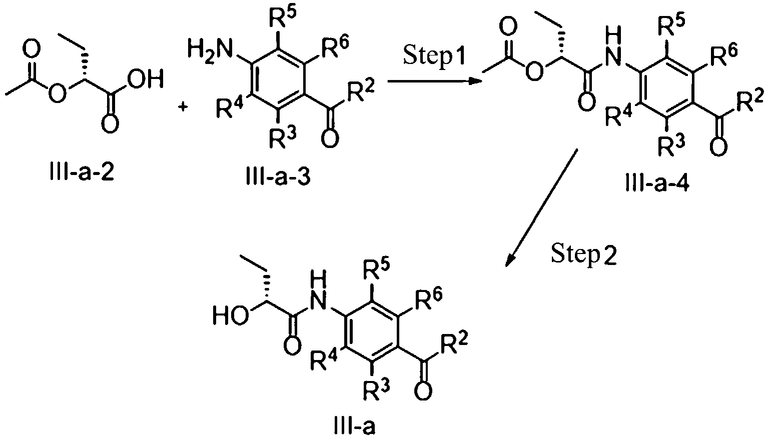
wherein, R², R³, R⁴, R⁵, and R⁶ are defined the same as in any of the foregoing claims;
Step 1: the compound of Formula (III-a-2) is subjected to a condensation reaction with the compound of Formula (III-a-3) to obtain the compound of Formula (III-a-4);
Step 2: the compound of Formula (III-a-4) is hydrolyzed under a basic condition to obtain the compound of formula (III-a).

11. The method according to claim 10, wherein the condensation reaction in step 1 comprises the following reaction conditions:
the condensation reaction includes a condensing agent, and the condensing agent comprises T3P or DPP-Cl;
the condensation reaction includes an organic base, and the organic base comprises triethylamine, pyridine, tetramethylguanidine, DBU, or DIPEA;
the solvent for the condensation reaction is an organic solvent; preferably, the organic solvent comprises any one or more of tetrahydrofuran, 2-methyltetrahydrofuran, isopropanol, ethanol, acetone, DMF, acetonitrile, and ethyl acetate;
optionally, the condensation reaction temperature is 0-60 °C;
optionally, the condensation reaction time is 1-10 h;
and/or the hydrolysis reaction in step 2 comprises the following reaction conditions:
the hydrolysis reaction includes an inorganic base, and the inorganic base includes potassium carbonate, sodium carbonate, cesium carbonate, potassium bicarbonate, or sodium bicarbonate;
the solvent for the hydrolysis reaction is a mixed solvent of an organic solvent and water, and the volume ratio of organic solvent to water in the mixed solvent is 1:1-10;
optionally, the molar ratio of the compound of Formula (III-a-4) to the inorganic base in the hydrolysis reaction is 1:1-10;
optionally, the hydrolysis reaction temperature is 0-60 °C;
optionally, the condensation reaction time is 1-10 h.

12. Use of the intermediate of any one of claims 1-6, or a pharmaceutically acceptable salt thereof, as a standard or a reference, or in the preparation of an oxopyridine compound of Formula (I) , or in the preparation of a medicament for the treatment or prophylaxis of vascular and arterial diseases.

13. A method for preparing an oxopyridine compound of Formula (I), comprising reacting an intermediate of Formula (II) or a pharmaceutically acceptable salt thereof with a compound of Formula (VI) to obtain a compound of Formula (I); 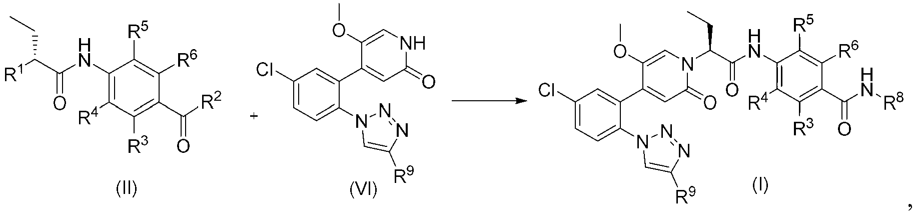 in Formula (II):
R¹ is selected from TsO-, X, and R⁷O-; wherein Ts is p-toluenesulfonyl, and X is selected from F, Cl, Br, and I;
R² is selected from -NHR⁸, and -OC(CH₃)₃;
R³, R⁴, R⁵, and R⁶ are independently selected from hydrogen, halogen, alkoxy, and haloalkyl;
R⁷ is selected from trifluoromethanesulfonyl, methanesulfonyl, p-nitrobenzenemethanesulfonyl and benzenesulfonyl;
R⁸ is selected from hydrogen, alkyl and cycloalkyl;
R⁹ is selected from fluoro, chloro and trifluoromethyl;
provided that Formula (II) is not

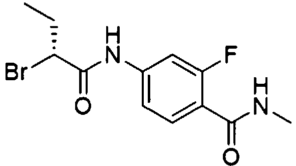

14. The method for preparing an oxopyridine compound of Formula (I) according to claim 13, wherein the method comprises:
when R² of Formula (II) is -OC(CH₃)₃, the intermediate of Formula (II) has the structure of Formula (II-a) and the method comprises the following steps: 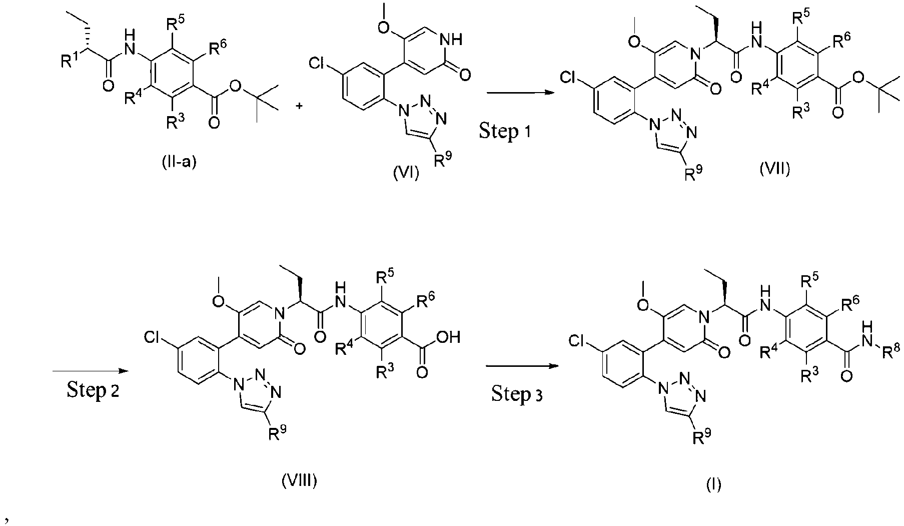
wherein,
R¹ is selected from TsO-, X, and R⁷O-; Ts is p-toluenesulfonyl, and X is selected from F, Cl, Br, and I;
R³, R⁴, R⁵, and R⁶are independently selected from hydrogen, halogen, alkoxy, and haloalkyl;
R⁷ is selected from trifluoromethanesulfonyl, methanesulfonyl, p-nitrobenzenemethanesulfonyl and benzenesulfonyl;
R⁸ is selected from hydrogen, alkyl and cycloalkyl;
R⁹ is selected from fluoro, chloro and trifluoromethyl;
Step 1: the intermediate of Formula (II-a), or a pharmaceutically acceptable salt thereof, is reacted with the compound of Formula (VI) to give the compound of Formula (VII);
Step 2: the compound of Formula (VII) is hydrolyzed to give the compound of Formula (VIII);
Step 3: the compound of Formula (VIII) is subjected to a condensation reaction to obtain the compound of Formula (I).

15. The method for preparing an oxopyridine compound of Formula (I) according to claim 13, wherein the method is selected from Method 1, 2 or 3;
Method 1: the method comprises obtaining the compound of Formula (I) by the reaction of the intermediate of Formula (III), or a pharmaceutically acceptable salt thereof with the compound of Formula (VI), 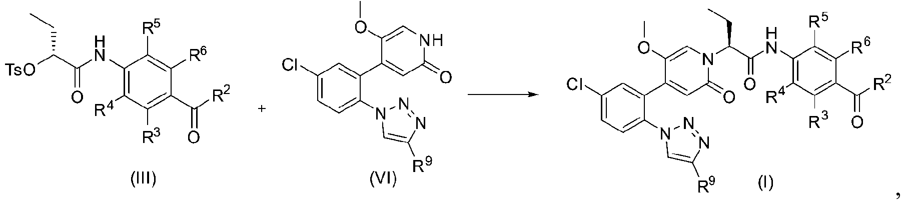 wherein,
R² is selected from -NHR⁸, and R⁸ is selected from hydrogen, alkyl and cycloalkyl;
R³, R⁴, R⁵, and R⁶ are independently selected from hydrogen, halogen, alkoxy, and haloalkyl;
R⁹ is selected from fluoro, chloro and trifluoromethyl;
Method 2: 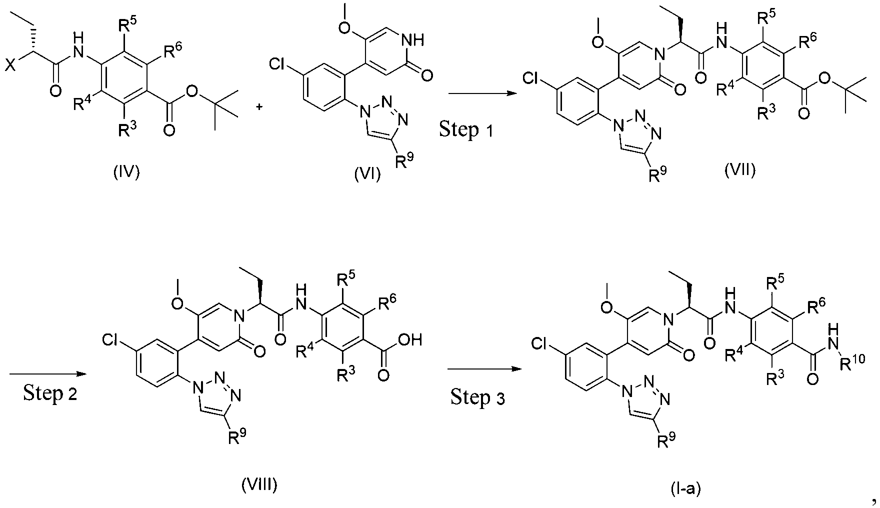 wherein,
R¹⁰ is selected from alkyl, cycloalkyl, and a deuterated alkyl or cycloalkyl;
R³, R⁴, R⁵, and R⁶ are independently selected from hydrogen, halogen, alkoxy, and haloalkyl;
R⁹ is selected from fluoro, chloro and trifluoromethyl;
X is selected form F, Cl, Br, and I;
Step 1: the intermediate of Formula (IV), or a pharmaceutically acceptable salt thereof, is reacted with the compound of Formula (VI) to give the compound of Formula (VII);
Step 2: the compound of Formula (VII) is hydrolyzed to give the compound of Formula (VIII);
Step 3: the compound of Formula (VIII) is subjected to a condensation reaction to obtain the compound of Formula (I-a),
Method 3: 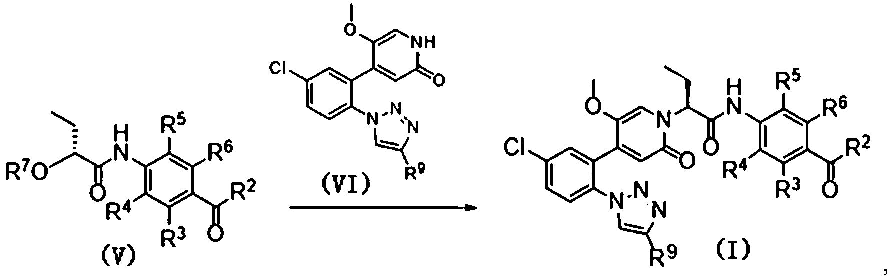
the intermediate of Formula (V) and the compound of Formula (VI) undergo nucleophilic substitution under a basic condition to obtain the compound of Formula (I);
wherein,
R⁹ is selected from fluoro, chloro and trifluoromethyl;
R² is selected from NHR⁸, and R⁸ is selected from hydrogen, alkyl and cycloalkyl;
R³, R⁴, R⁵, and R⁶ are independently selected from hydrogen, halogen, alkoxy, and haloalkyl;
R⁷ is selected from trifluoromethanesulfonyl, methanesulfonyl, p-nitrobenzenemethanesulfonyl and benzenesulfonyl.

16. The method for preparing an oxopyridine compound of Formula (I) according to claim 15, wherein Method 1 and/or Method 3 comprises the following reaction conditions:
the reaction condition includes a base, and the base is selected from organic bases and inorganic bases;
the reaction solvent is selected from organic solvents;
optionally, the molar ratio of the compound of Formula (III) or Formula (V) to the base is 1:1-3;
optionally, the reaction temperature is 0-60 °C;
optionally, the reaction time is 1-10 h;

17. The method for preparing an oxopyridine compound of Formula (I) according to claim 16, wherein
the base includes any one of sodium carbonate, potassium carbonate, cesium carbonate, potassium bicarbonate, potassium phosphate, dipotassium hydrogen phosphate, potassium dihydrogen phosphate, sodium bicarbonate, lithium hydroxide, sodium hydroxide, potassium hydroxide, tetramethylguanidine, triethylamine, DBU, DIPEA, and pyridine, or a mixture of two or more thereof;
the organic solvent comprises any one of tetrahydrofuran, 2-methyltetrahydrofuran, isopropanol, ethanol, acetone, DMF, and dioxane, or a mixture of two or more thereof;
optionally, the molar ratio of the compound of Formula (III) or Formula (V) to the base is 1:2;
optionally, the reaction temperature is 20-40 °C;
optionally, the reaction time is 4-6 h.

18. The method for preparing an oxopyridine compound of Formula (I) according to claim 15, wherein Method 2 comprises the following reaction conditions:
Step 1 comprises the following reaction conditions,
Step 1 includes a base, and the base is selected from organic bases and inorganic bases;
the solvent for Step 1 is selected from organic solvents, and the organic solvents comprise any one of isopropanol, ethanol, acetone, DMF, tetrahydrofuran, 2-methyltetrahydrofuran, and dioxane, or a mixture of two or more thereof.
optionally, the molar ratio of the compound of Formula (IV) to the base in Step 1 is 1:1-3;
optionally, the reaction temperature of Step 1 is 0-60 °C;
optionally, the reaction time of Step 1 is 1-10 h;
or Step 2 comprises the following reaction conditions,
Step 2 includes an acid, and the acid is selected from any one of hydrochloric acid, trifluoroacetic acid, sulfuric acid, phosphoric acid, acetic acid, and hydrobromic acid, or a mixture of two or more thereof;
the reaction solvent for Step 2 is selected from organic solvents; and the organic solvents comprise any one of isotetrahydrofuran, 2-methyltetrahydrofuran, dioxane, acetone, methanol, ethanol, isopropanol, and DMF, or a mixture of two or more thereof;
optionally, the molar ratio of the compound of Formula (VII) to the acid in Step 2 is 1:30;
optionally, the reaction temperature of Step 2 is -20 to 40 °C;
optionally, the reaction time of Step 2 is 1-8 h;
or Step 3 comprises the following reaction conditions,
Step 3 includes a base, and the base includes any one of triethylamine, DBU, DIPEA, tetramethylguanidine, pyridine, sodium carbonate, potassium carbonate, cesium carbonate, potassium bicarbonate, sodium bicarbonate, lithium hydroxide, sodium hydroxide, and potassium hydroxide, or a mixture of two or more thereof.
Step 3 includes a condensing agent and a ligand, and the condensing agent and ligand include any one of EDCI, HOBT, HATU, HBTU, DCC, CDI, T3P, DPP-Cl, HCTU, TBTU, and DMAP, or a mixture of two or more thereof;
the reaction solvent for Step 3 is selected from organic solvents, and the organic solvents comprise any one of isotetrahydrofuran, DCM, 2-methyltetrahydrofuran, dioxane, acetonitrile, acetone, ethanol, isopropanol, DMF, and DMAC, or a mixture of two or more thereof;
optionally, the molar ratio of the compound of Formula (VIII) to the base in Step 3 is 1:1-5;
optionally, the reaction temperature of Step 3 is 0-60 °C;
optionally, the reaction time of Step 3 is 1-10 h.
